Europäisches Patentamt

European Patent Office

Office européen des brevets

(11) Publication number: 0 234 592
A1

# EUROPEAN PATENT APPLICATION

(21) Application number: 87102830.4

(22) Date of filing: 27.02.87

(51) Int. Cl.³: **C 12 N 15/00**
C 12 N 5/00, C 12 P 21/00
//(C12N15/00, C12R1:07),
(C12N5/00, C12R1:19)

(30) Priority: 28.02.86 JP 43530/86
28.02.86 JP 43531/86

(43) Date of publication of application:
02.09.87 Bulletin 87/36

(84) Designated Contracting States:
CH DE FR GB LI

(71) Applicant: TEIJIN LIMITED
11 Minamihonmachi 1-chome Higashi-ku
Osaka-shi Osaka 541(JP)

(71) Applicant: RIKAGAKU KENKYUSHO
2-1 Hirosawa
Wako-shi Saitama-ken(JP)

(72) Inventor: Kitai, Kazuo
Teijin Musashino-ryo 3-5-18, Tamadaira
Hino-shi Tokyo(JP)

(72) Inventor: Nakamura, Satoshi
Teijin Tama apart. 122 3-18-4, Tamadaira
Hino-shi Tokyo(JP)

(72) Inventor: Horikoshi, Koki
4-39-8, Sakuradai
Nerima-ku Tokyo(JP)

(72) Inventor: Kudo, Toshiaki
1-21-20-606, Tairamachi
Meguro-ku Tokyo(JP)

(74) Representative: Vossius & Partner
Siebertstrasse 4 P.O. Box 86 07 67
D-8000 München 86(DE)

(54) Plasmid containing DNA fragment coding for human immunoglobulin G Fc region protein and use thereof for production of said protein.

(57) A plasmid containing a DNA region coding for human immunoglobulin G Fc regoin protein, a DNA region exhibiting a promoter function for controlling the expression of the protein, and a DNA region coding for a signal peptide; and a plasmid containing: (a) a DNA fragment comprising a DNA region coding for a human immunoglobulin G Fc region protein, a DNA region exhibiting a promoter function for controlling the expression of the protein, and a DNA region coding for a signal peptide and (b) a DNA fragment comprising a DNA region causing host cells to facilitate the extracellular secretion of the protein, and a DNA region exhibiting a promoter function for controlling the expression of the DNA region; as well as microbial cells transformed with the above-mentioned plasmid; and a process for production of the Fc region protein using the above-mentioned microbial cells.

<u>PLASMID CONTAINING DNA FRAGMENT CODING FOR</u>

<u>HUMAN IMMUNOGLOBULIN G FC REGION PROTEIN AND</u>

<u>USE THEREOF FOR PRODUCTION OF SAID PROTEIN</u>

BACKGROUND OF THE INVENTION

1.    Field of the Invention

The present invention relates to new recombinant plasmids containing a DNA fragment coding for a human immunoglobulin G Fc region protein, microbial cells transformed with the plasmid, and a process for production of the human immunoglobulin G Fc region protein wherein the protein is transported to the periplasmic space or secreted out of the cells.

2.    Description of the Related Art

An antibody is a protein present in the body fluid of vertebrates and capable of specific binding to an antigen.  Proteins with a structure and functions related to the antibody proteins are generally designated as immunoglobulins.  These immunoglobulins (abbreviated as Ig hereinafter) are classified into five classes, i.e., IgG, IgA, IgM, IgE, and IgD.

Among these immunoglobulins, IgG plays an important role in the biological defense against bacteria and viruses.

Human IgG comprises two heavy chains (abbreviated hereinafter as H chains) and two light chains (abbreviated hereinafter as L chains). The H chain and    L chain are linked via a disulfide bond, the two H chains are linked via several disulfide bonds. When a human IgG molecule is subjected to the action of a protease such as papain, the molecule is cleaved at approximately the center thereof into three fragments; two fragments having an antigen-binding activity (Fab region protein) and the other fragment having no antigen-binding activity but can be easily crystallized (Fc region protein).  The Fab region protein comprises an entire L chain and an amino terminal half of the H chain. The Fc region protein, on the other hand, comprises two fragments, each of which is a carboxy terminal half of the H chain, and these fragment are comprised of a hinge region, a $C_H2$ domain and a $C_H3$ domain, in that order, and links to the other fragment via several disulfide bonds at the hinge regions. The Fc region has a function as an effector.

As described above, since the IgG plays an important role in biological defense, and is present in a large amount in blood, a human IgG rich γ-globulin fraction is obtained from human blood and used in a partially modified form as an immunological agent for administration to patients with symptoms of a serious nature. That is, the γ-globulin-containing pharmaceuticals are used as a supplement in hypogammaglobulin-emia, and for the prevention and treatment of viral diseases. Recently, it has been suggested that the γ-globulin preparation is effective for the treatment of idiopathic chromobopenic purpura (ITP) (see, P. Imbach et al., Lancet, 1, 1228, 1981); and especially, it was suggested that the Fc region is important in the above-mentioned application (see, Boku et al., Rinsho Meneki, 15, 76, 1983). Moreover, immuncomplexes deposited on glomerulum in the case of systemic lupus erythematosus (SLE) were reported to be dissolved by adding human IgG Fc region protein (see, Kawasumi, Rinsho Meneki 16, 240, 1984).

Nevertheless, although the γ-globulin preparations and the Fc region protein derived therefrom are useful as therapeutic agents for autoimmune diseases such as ITP and SLE, as mentioned above, such immunological preparations have certain disadvantages. For example, since the preparations are prepared from human blood, which is difficult to obtain in a large amount as one lot, many lots of blood must be pooled to obtain a substantial amount of the preparation. This gives rise to the problem of a diversity in the quality of the preparations, and a higher risk of viral contamination.

Therefore, it is much more desirable to produce the human immunoglobulin G Fc region protein by gene technology. The human IgG H chain is classified into four subclasses, i.e., $\gamma_1$ chain, $\gamma_2$ chain, $\gamma_3$ chain, and $\gamma_4$ chain. Among these subclasses, for the $\gamma_2$ and $\gamma_4$ chains, U. Krawinkel et al., EMBO J. 1, 403 (1982) disclose a partial nucleotide sequence of a $\gamma_2$ chain gene and a $\gamma_4$ chain gene; and for the $\gamma_1$ chain, J.W. Ellison et al., Nucleic Acids Res. 10, 4071 (1982) disclose a partial nucleotide sequence of a $\gamma_1$ chain gene. However, such reports do not disclose a DNA fragment capable of expressing a human IgG H chain gene.

In many kinds of microorganisms including Escherichia coli, cytoplasm is enveloped by an inner membrane and an outer membrane, and a space called the periplasmic space is formed between these two membranes. In most case wherein a desired polypeptide is produced by gene technology, the polypeptide produced in a host cell accumulates in the cytoplasm because the polypeptide cannot pass through the inner membrane. In limited cases, the expressed polypeptide is transported through the inner membrane and accumulates in the periplasmic space; and in more limited cases, the polypeptide is extracellularly secreted.

In the production of a desired polypeptide, the accumulation of the polypeptide in the cytoplasm has many disadvantages. For example, a feedback mechanism would repress the production of the desired polypeptide; when a desired polypeptide is toxic to host cells, the polypeptide accumulated in the cytoplasm would adversely affect the host cell; and if a desired product is susceptable to an intracellular protease, the product would be hydrolyzed by the protease. Moreover, even if these adverse conditions do not exist, the product cannot accumulate in the host cell in a volume more than the volume of the host cell. Moreover, since the host cell can produce many kinds of proteins other than a desired product, during a purification process for the desired product, it is very difficult to isolate the desired product from the undesirable proteins.

If a desired product is transported to the periplasmic space, the feedback mechanism would not be triggered by the accumulation of the product, and the toxic action of the product against the host cell would be reduced. Moreover, the desired product may be purified and isolated relatively easily, because the product accumulated in the periplasmic space can be released by a means such as osmotic shock, which does not release cellular components present in the cytoplasm.

If a desired product is extracellularly secreted, in addition to the above-mentioned advantages, the desired product may be produced without limitation, because the product is diluted in a cultured broth having a volume far greater than the total volume of the producer cells. Moreover, since the product accumulates in a supernatant, it is very easy to purify and isolate the product at a reduced cost during industrial production thereof.

Generally, secretory proteins are produced in a form wherein the protein has at the amino terminal thereof an additional peptide consisting of about 20 to 40 amino acids. This peptide is called the signal peptide or signal sequence. The protein having a signal peptide is then transported through the cell membrane. During this process the signal peptide is clipped off by a signal peptidase providing the secretion of the protein having no signal peptide. Since it is believed that this mechanism for the transportation of the protein is fundamentally common throughout higher organisms and lower organisms, including microorganisms such as E. coli, it is expected that, by attaching a signal peptide to a protein which cannot be secreted in its native form, the protein can be transported through the cell membrane; in the case of E. coli, the inner membrane. In such a case, the protein should accumulate in the periplasmic space.

Recently, several of the present inventors, - using the plasmid vector pMB9 (R.L. Rodriguez et al., Molecular Mechanisms in the Control of Gene Expression, ICN-UCLA, Symp. Mol. Cell Biol., ed. D.P. Nierlich et al., V, 471, Academic Press Inc., New York, 1976), successfully cloned a penicillinase gene derived from chromosomal DNA of alkalophilic Bacillus No. 170 in E. coli (T. Kudo et al., J. Bacteriol., 156, 949, 1983). This penicillinase gene contained a promoter region, a signal peptide-coding region, and a region coding for a mature penicillinase protein.

When the above-mentioned E. coli was cultured, the penicillinase protein was observed to be extracellularly secreted, revealing that, by activating the Kil gene which has no promoter in itself and is present on the plasmid pMB9, by a promoter region (Ex promoter) present near the penicillinase gene in the DNA fragment derived from the alkalophilic Bacillus No. 170, the permeability of E. coli outer membrane was increased (Horikoshi, Gendai Kagaku, 176, 56, 1985).

Note, the above-mentioned alkalophilic Bacillus strain No. 170 was deposited with the Fermentation Research Institute, Agency of Industrial Science and Technology (1-3, Higashi 1-chome, Yatabe-cho, Tsukuba-gun, Ibaraki-ken, Japan) under the Budapest Treaty as FERM BP-469.

SUMMARY OF THE INVENTION

On the basis of the above-mentioned finding, the present inventors successfully constructed plasmids which can transport human immunogrobulin G Fc region proteins into the periplasmic space in E. coli cells, and plasmid which can extracellularly secrete such proteins. They found that a major portion of the human immunoglobulin G Fc region protein thus secreted was a

- 6 -

0234592

dimer consisting of two polypeptide chains linked via disulfide bonds.

Therefore, the present invention provides plasmids containing a DNA region coding for a human immunoglobulin G Fc region protein, a DNA region exhibiting a promoter function for controlling expression of the protein, and a DNA region coding for a signal peptide.

The present invention also provides microbial cells transformed with the above-mentioned plasmids.

The present invention further provides a process for production of human immunoglobulin G Fc region protein comprising culturing the transformed microbial cells until the protein accumulates in the periplasmic space, and recovering the human immunoglobulin G Fc region protein from the periplasmic space.

Moreover, the present invention provides plasmids containing:

(a) a DNA fragment comprising a DNA region coding for a human immunoglobulin G Fc region protein, a DNA region exhibiting promoter function for controlling expression of the protein, and a DNA region coding for a signal peptide; and

(b) a DNA fragment comprising a DNA region causing host cells to facilitate extracellular secretion of said protein, and a DNA region exhibiting a promoter function for controlling expression of the DNA region.

The present invention also provides microbial cells transformed with the above-mentioned plasmid.

The present invention further provides a process for production of human immunoglobulin G Fc region protein comprising culturing the transformed microbial cells until the protein accumulates outside the cells, and recovering the human immunoglobulin G Fc region protein from the culture broth.

BRIEF DESCRIPTION OF THE DRAWINGS

Figure 1 represents a part of a nucleotide sequence in plasmid pPS-FC which can express a human immunoglobulin G Fc region protein and which can transport the protein into the periplasmic space, and a corresponding amino acid sequence of alkalophilic Bacillus No. 170 penicillinase signal peptide and the Fc region protein, wherein an amino acid sequence from the first Met to 31st Ser forms the signal peptide and the amino acid sequence from the 32nd Thr to 254th Lys forms the Fc region protein;

Fig. 2 represents a nucleotide sequence of a Kil gene present in plasmid pMB9;

Fig. 3 represents a nucleotide sequence of an Ex promoter region present in the chromosomal DNA of alkalophilic Bacillus No. 170;

Fig. 4 represents a restriction enzyme cleavage map (A) of a phage clone containing a human immunoglobulin G gene, and restriction enzyme cleavage maps (B) and (C) of sub-clones pTJ1B and pTJ5 containing an Fc region gene;

Fig. 5 represents a process for construction of plasmid pFC70 containing a $C_H3$ domain gene;

Fig. 6 represents a process for construction of plasmid pFC77 containing a $C_H2-C_H3$ domain gene;

Figs. 7, 8, and 9 represent processes for the construction of Fc region gene intracellular accumulation type plasmids pFC203, pFC211, and pFC362, respectively;

Fig. 10 represents a process for isolation and purification of plasmid DNA from E. coli cells;

Fig. 11 represents a process for cloning penicillinase gene of alkalophilic Bacillus No. 170;

Figs. 12, 13, 14, and 15 represent a process for the construction of plasmids pEAP3, pEAP6, pEAP7, pHAP7ΔH, pEAP7ΔCCH, pEAP7ΔCH, pEAP8 and p329EXK, all containing a DNA region carrying information relating to an extracellular secretion of the expression product;

Fig. 16 represents a process for construction of plasmid pCM71 containing a chloramphenicol acetyl transferase gene;

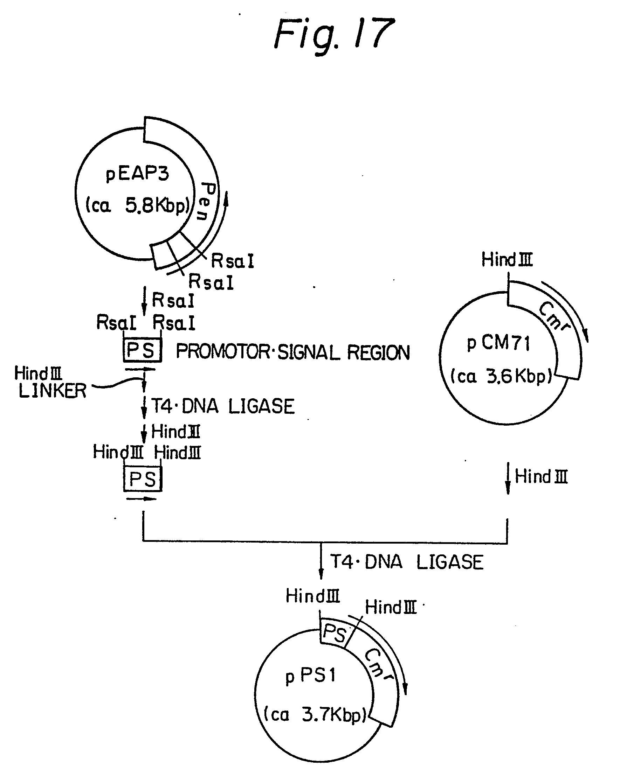

Figs. 17 and 18 represent a process for construction

of plasmids pPS1, pPS1ΔH, and p329P8, all containing a promoter region and signal peptide region of the penicillinase gene;

Fig. 19 represents a process for construction of plasmids pSEC-FC and pSEC-FCC containing an Fc region gene having a joint for linking with a signal peptide region;

Fig. 20 represents a process for construction of plasmid pPS-FC which can express the Fc region gene and transport the expressed product to the periplasm space;

Figs. 21 and 22 represent processes for construction of plasmids pEXFC10 and pEXFC100 respectively, which can express an Fc region gene and extracellularly secrete the expressed product;

Fig. 23 represents a result of the secretion of expressed products from various transformants; and

Fig. 24 represents a result of electrophoresis for confirming a dimer structure of the Fc region protein expressed by the present plasmid.

DESCRIPTION OF THE PREFERRED EMBODIMENT

In the first embodiment of the present invention, the present plasmid contains a DNA region coding for a human immunoglobulin G Fc region protein, a DNA region exhibiting a promoter function for controlling expression of the above-mentioned protein, and a DNA region coding for a signal peptide.

In a preferred embodiment, the DNA region coding for the human immunoglobulin G Fc region protein is derived from cells producing human immunoglobulin G, for example, human lymphoma cell line ARH77, and the DNA region exhibiting the promoter function for controlling the expression of the peptide and the DNA region coding for the signal peptide are derived from the chromosomal DNA of alkalophilic Bacillus No. 170.

In the second embodiment, the present plasmid contains, in addition to the above-mentioned DNA regions of the first embodiment, a DNA region causing host cells to facilitate extracellular secretion of the expressed protein, and a DNA region exhibiting a promoter function for controlling the expression of said DNA region.

In a preferred embodiment, the DNA region causing host cells to facilitate the extracellular secretion of the expressed protein is derived from plasmid mPB9, and the DNA region exhibiting the promoter function for controlling the expression of said DNA, is derived from the chromosomal DNA of alkalophilic Bacillus No. 170.

A. Cloning of human immunoglobulin G Gene

(a) Preparation of a human chromosomal DNA and a gene library

Cells producing human IgG, for example, human lymphoma cell line ARH77 (see K.H. Burk et al. J. Cancer Res. 38, 2508, 1978), are cultured under appropriate conditions, for example, at a temperature of 37°C and at a carbon dioxide concentration of 5%, and the resulting cells are collected by centrifugation. The cells are lysed with a protease such as protease K in the presence of a detergent such as sodium dodecyl sulfate (SDS). The lysate is extracted by, for example, phenol, to eliminate proteins, and the human chromosomal DNA is obtained.

The chromosomal DNA thus obtained is then digested with a restriction enzyme such as Eco RI to obtain DNA fragments, which are then ligated to a phage vector such as Charon 4A vector (F.R. Blattner et al., Science, 196, 161, 1977), and the ligation products are subjected to in vitro packaging (A. Becker et al., Proc. Natl. Acad. Sci. USA, 72, 581, 1975) to obtain a human gene library. If a restriction enzyme other than Eco RI is used, or another phage vector with no Eco RI site as a cloning site is used, an appropriate linker DNA may be used to prepare the gene library.

(b) Preparation of restriction enzyme cleavage map of subclone

The phages in the gene library are transfected into a host such as E. coli LE 392 (ATCC 33572) to form plaques, which are then screened by plaque hybridization (W.P. Benton et al. Science, 196, 180, 1977) to select the desired clones. The probe used for the screening is, for example, the human immunoglobulin H chain J region (which is a part of the Fab region and is present between a variable region having an antigen binding activity and a constant region having an effector function) gene labeled $^{32}$P according to nick translation (P.W.J. Rigby et al. J. Mol. Biol. 113, 237, 1977), or a synthetic oligonucleotide predicted to correspond to a part of an amino acid sequence of the human IgG Fc region protein, and labeled with $^{32}$P as above-mentioned.

For the DNA fragment of clones positive in the above-mentioned plaque hybridization, restriction enzyme cleavage site maps are prepared and DNA fragments derived from human chromosomes are subcloned to a plasmid vector such as pBR322 (F. Bolivar et al. Gene, 2, 95, 1977).

Insert DNA of the resulting subclones is sequenced according to the Maxam-Gilbert method (A.M. Maxam et al. Methods Enzymol. 65, 499, 1980), or the dideoxy chain termination method using M13 phage (J. Messing et al. Nucleic Acids Res. 9, 309, 1981) and the like, to confirm the presence of the human IgG Fc region gene.

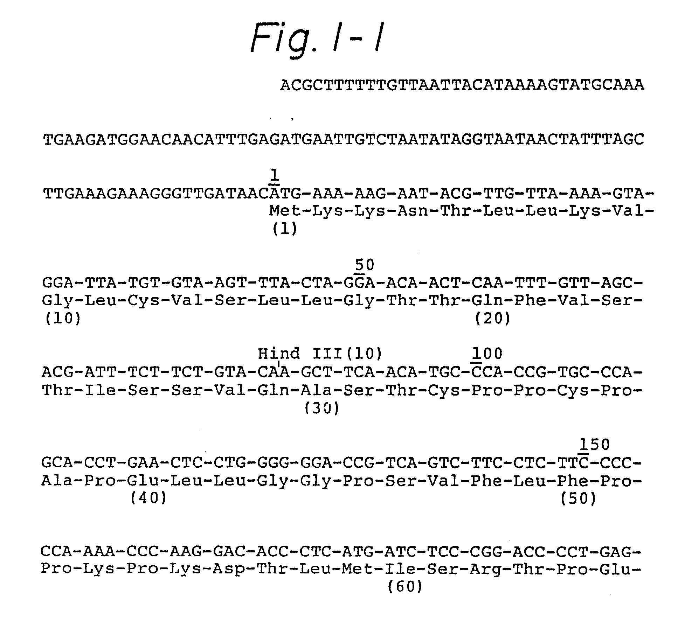

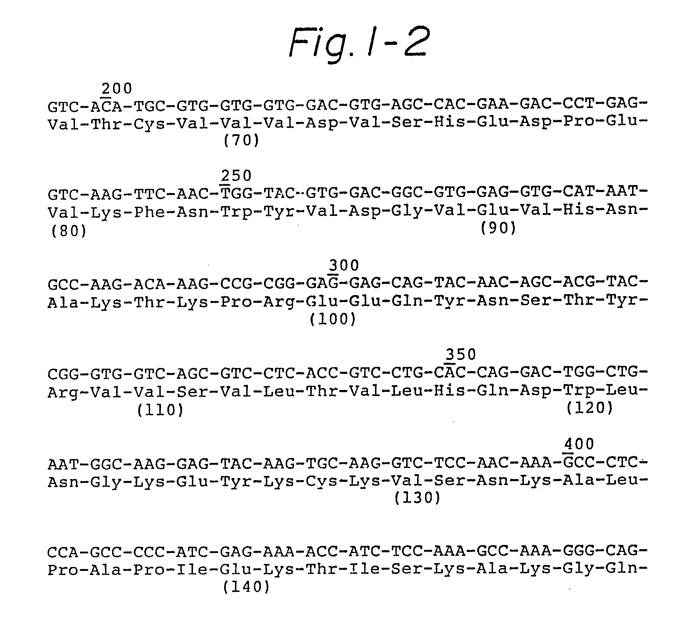

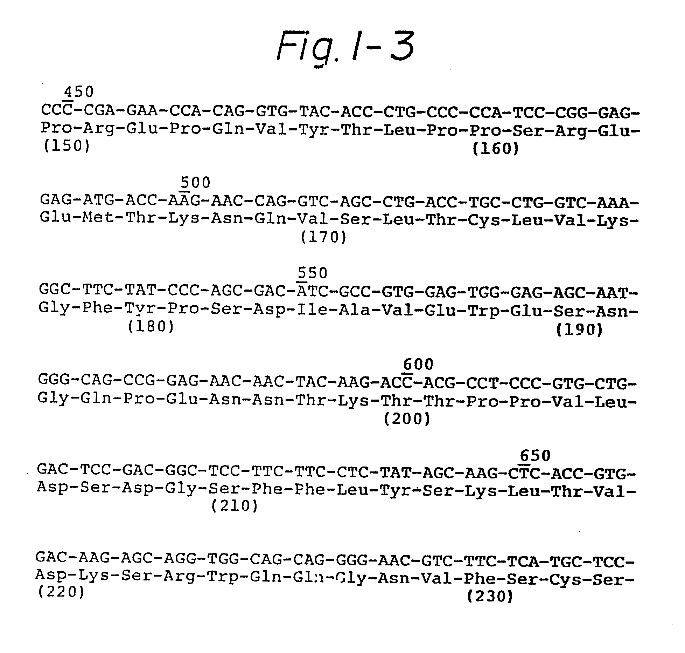

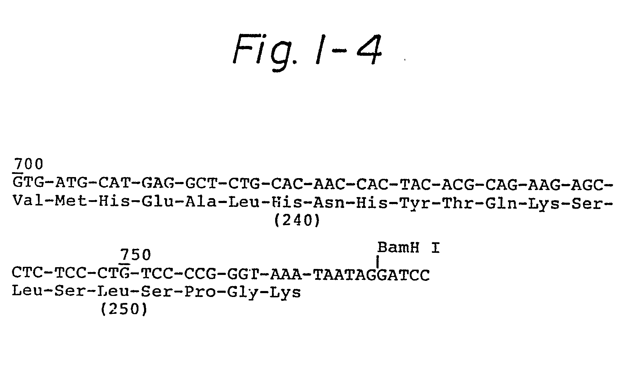

Figs. 1-1 to 1-4 represent an example of a DNA sequence and a corresponding amino acid sequence of a human immunoglobulin G Fc region protein.

(c)　Preparation of DNA fragment

Since the human IgG Fc region protein gene thus obtained is of human chromosome origin, it contains introns and, therefore, cannot be expressed in microorganisms. Therefore, the Fc region gene is cleaved with appropriate restriction enzymes to eliminate entirely the intron regions. If a part of the exon is deleted by the cleavage with the restriction enzymes, a chemically synthesized linker is used to repair the deleted part and to link adjacent exons. Similarly, by using synthetic oligonucleotides, more than one translation stop codon in tandem (TGA, TAG or TAA) can be attached, in reading frame with the Fc region gene at the 3'-end thereof, to enhance the efficiency of gene expression. The thus-obtained Fc region gene without any introns can be joined to a translation start codon upstream of and in reading frame with the gene. Moreover, the Fc region gene can be linked downstream from an appropriate promoter and Shine-Dalgarno (SD) sequence to form an expression gene. Useful promoters include the tryptophan operon promoter (trp promoter), lactose operon promoter (lac promoter), tac promoter, $P_L$ promoter, $I_{pp}$ promoter, and the like. The trp promoter and tac promoter are especially preferable. For efficient expression of the Fc region gene, the Fc region gene is preferably linked to a promoter, SD sequence, and a translation start codon positioned upstream thereof, and to translation stop codons positioned downstream thereof. Most preferably, a promoter, an SD sequence, a translation start codon, an Fc region gene, and translation stop codons are linked, in this order.

(d) Construction of recombinant plasmids and recombinant microorganisms

The expression plasmid can be constructed by inserting the expression-type human IgG Fc region gene into an appropriate plasmid vector such as pBR322. The expression plasmids according to the present invention preferably include pFC203, pFC211, pFC361, and pFC362.

B. Cloning of penicillinase gene of alkalophilic Bacillus No. 170

Alkalophilic Bacillus No. 170 (FRM BP-467) is cultured under appropriate conditions, such as at 30°C with shaking, and cultured cells are collected by, for example, centrifugation. The cells thus prepared are extracted in a conventional manner by, for example, a phenol extraction method, to obtain chromosomal DNA.

The DNA thus obtained is partially digested with an appropriate restriction enzyme, such as Eco RI, and the DNA fragment is inserted into the Eco RI restriction site of, for example, plasmid pMB9 to obtain a recombinant plasmid containing an DNA insert derived from chromosomal DNA of alkalophilic Bacillus No. 170. The recombinant plasmid is inserted into, for example, E. coli HB101 (ATCC 33694), in a known manner, for example, by the $CaCl_2$ method (M.V. Norgard et al., Gene, 3, 279 (1978), and the transformants are screened for ampicillin and tetracycline resistance to obtain a recombinant plasmid containing a DNA region carrying information responsible for the extracellular secretion of penicillinase, for example, plasmid pEAP1.

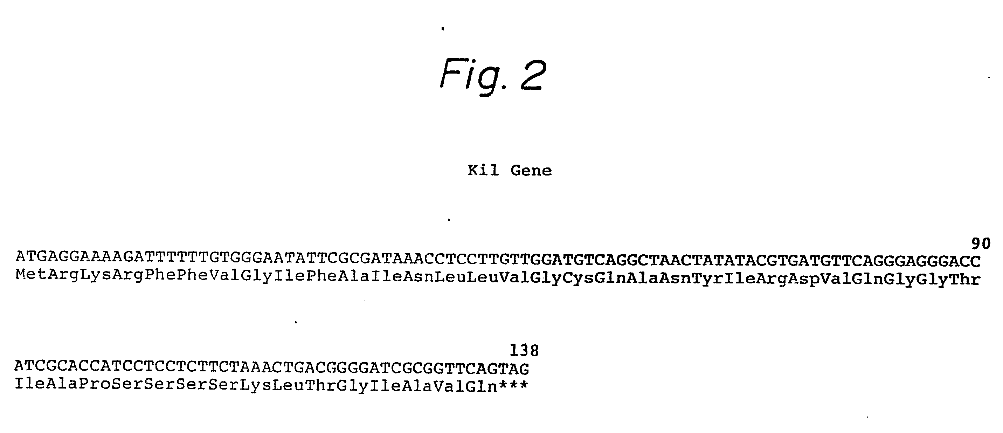

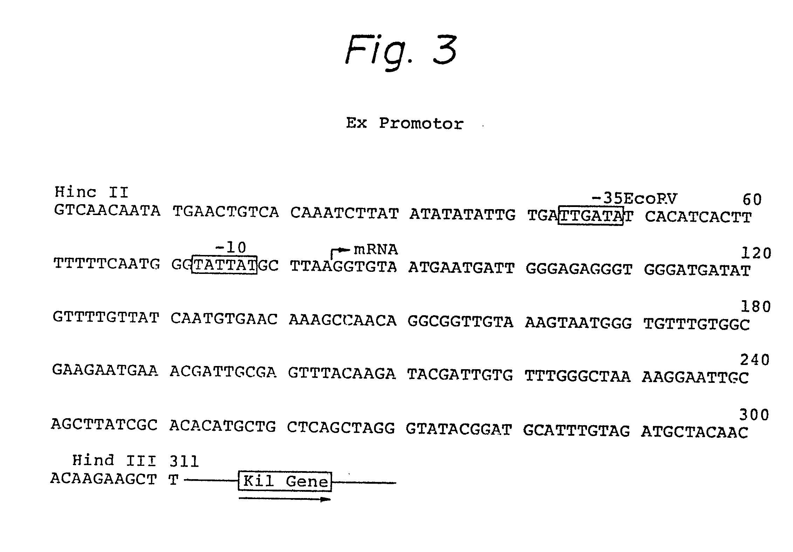

The plasmid thus obtained is then sequenced according to, for example, the above-mentioned Maxam-Gilbert method or the dideoxy chain termination method using M13 phage, to clarify the structures of the penicillinase gene promoter region, signal peptide region, mature peptide coding region, and Ex promoter region responsible for the extracellular excretion of penicillinase, all derived from chromosomal DNA of alkalophilic Bacillus No. 170, as well as a Kil gene derived from plasmid pMB9. Figure 1 represents a nucleotide sequence including a penicillinase gene promoter region, and signal peptide coding region and a corresponding amino acid sequence. Figure 2 represents a nucleotide sequence of the Kil gene derived from plasmid pMB9. Figure 3 represents a nucleotide sequence of an Ex promoter derived from alkalophilic Bacillus No. 170 and a corresponding amino acid sequence.

Starting from the recombinant plasmid containing a penicillinase gene and a DNA region carrying information responsible for the extracellular secretion of penicillinase, recombinant plasmids containing an entire DNA region carrying information responsible for the extracellular secretion, which region comprises the Ex promoter region derived from alkalophilic Bacillus No. 170 and the Kil gene derived from plasmid pMB9 as well as a part of or the entire penicillinase region are obtained, according to a method using spontaneous deletion or an artificial method using modification by enzymes such as S1 nuclease, DNA polymerase, and the like, and synthetic oligonucleotide. Such plasmids preferably include plasmids pEAP3, pEAP6, pEAP7, pEAP7ΔH, pEAP7ΔCCH, pEAP7ΔCH, pEAP8, and p329EXK.

Moreover, the above-mentioned recombinant plasmid containing a penicillinase gene and a DNA region carrying information responsible for the extracellular secretion of penicilinase is digested with an appropriate restriction enzyme to obtain a DNA fragment containing a promoter region and signal peptide region of penicillinase gene. This DNA fragment is cloned, if necessary via a synthetic oligonucleotide linker, into an appropriate plasmid vector, for example, hybrid plasmid pCM71 derived from plasmids pCM1 (T.J. Close and R. Rodriguez, Gene, 20, 305, 1982) and pCM7 (T.J. Close and R. Rodriguez, Gene, 20, 305, 1982), to obtain a plasmid containing a promoter region and a signal peptide region of the penicillinase gene. As the plasmids containing a promoter region and signal peptide region of the penicillinase gene, plasmids pPS1, pPS1ΔH, and p329PS are preferably used.

C. Construction of secretion type plasmids

The above-prepared plasmid for intracellular accumulation of the human immunoglobulin G Fc region protein, such as pFC362, is digested with an appropriate restriction enzyme to delete an original promoter region which cannot direct the secretion of the expression product, and a synthetic oligonucleotide linker is inserted in the deleted region to obtain an intermediate plasmid. The linker is used to link an appropriate promoter region and signal peptide region such as the penicillinase gene signal peptide region derived from alkalophilic <u>Bacillus</u> No. 170 to a human immunoglobulin G Fc region gene. As the intermediate plasmid, plasmids pSEC-FC and pSEC-FCC are preferably used.

Next, into the intermediate plasmid, a DNA fragment comprising an appropriate promoter region and a DNA fragment comprising an appropriate signal peptide region are inserted to obtain a plasmid wherein the human immunoglobulin G Fc region gene is linked downstream from the promoter region and signal peptide region. The above-mentioned DNA fragment comprising the promoter region and a DNA fragment comprising the signal peptide region are obtained by digesting a plasmid containing such a promoter region and signal peptide region with an appropriate restriction enzyme.

As an origin of such a promoter region and signal peptide region, an <u>E. coli</u> β-lactamase gene, <u>E. coli</u> alkaline phosphatase gene, <u>E. coli</u> lipoprotein gene, <u>Bacillus</u> <u>subtilis</u> penicillinase gene, <u>Bacillus</u> <u>subtilis</u> protease gene, yeast α-factor gene, alkalophilic <u>Bacillus</u> No. 170 penicillinase gene, alkalophilic <u>Aeromonas</u> No. 212 xylanase gene, or alkalophilic <u>Bacillus</u> No. N-4 cellulase gene are used. Preferably, an alkalophilic <u>Bacillus</u> No. 170 penicillinase gene is used. A plasmid wherein an appropriate promoter region, an appropriate signal peptide region, and a human immunoglobulin G Fc region gene are linked, in this order, is most preferable. By using such a plasmid, a human immunoglobulin G Fc region protein can be transported to the periplasmic space (periplasmic transportation). As the periplasmic transportation type plasmid, preferably pPS-FC is used.

Moreover, by combining the various plasmids described above, a plasmid containing both a DNA region comprising an appropriate promoter region, signal peptide region, and a human immunoglobulin G Fc region gene linked downstream from the signal peptide region, and a DNA region carrying information responsible for the extracellular secretion and comprising an Ex promoter region derived from alkalophilic _Bacillus_ No. 170 and Kil gene derived from pMB9 plasmid is obtained. The use of this plasmid makes possible the extracellular secretion of the human immunoglobulin G Fc region protein. As the extracellular secretion type plasmid, preferably plasmids pEXFC10 and pEXFC100 are used.

Note, according to the present invention, the above-mentioned promoter region, signal peptide region, human immunoglobulin G Fc region gene, Ex promoter region, and/or Kil gene can be replaced by other DNA regions having corresponding biological functions but modified by a replacement of one or more nucleotides, deletion of one or more nucleotides, insertion of one or more nucleotides, or inversion of a nucleotide sequence, or other kinds of mutations.

D. Production of human immunoglobulin G Fc region protein

To produce the human immunoglobulin G Fc region protein, an intracellular accumulation type plasmid, periplasmic transportation type plasmid or extracellular secretion type plasmid, prepared as described above, is used to transform an appropriate host to obtain a transformant, and the transformant is then cultured. As a host, microorganisms belonging to the genus _Escherichia_, such as _E. coli_ HB101, _E. coli_ C600-R⁻M⁻, _E. coli_ DP-supF, _E. coli_ X1776, _E. coli_ LE392, and the like conventionally used in the genetic engineering field, are preferably used. _E coli_ HB101 and _E. coli_ C600R⁻M⁻ are particularly preferable.

The transformant thus obtained is cultured in a manner known per se. The culture medium is any medium suitable for culturing the transformed host microorganism described above, and for the production of the human immunoglobulin G Fc region protein. For example, M9 medium (T. Maniatis et al., Molecular Cloning p440, Cold Spring Harbor Laboratory, New York, 1982), LB medium (T. Maniatis et al., Molecular Cloning, p440, Cold Spring Harbor Laboratory, New York, 1982), BPB medium (Difco), Nutrient Agar medium or the like is used as a basal medium. In addition to a carbon source and nitrogen source, if necessary, nutrients such as amino acids, vitamines or the like may be added to the basal medium. Moreover, depending on the selective maker in the expression plasmid, an appropriate amount of antibiotics may be added to the medium to stabilize the plasmid in the host cells.

Culturing conditions such as the pH, temperature, and oxygen supply level, are controlled so that they are preferable for culturing host cells. The pH value is preferably 5 to 8, most preferably 7. When a recombinant microorganism containing an intracellular accumulation type plasmid is cultured, an agent such as isopropyl-β-D-thiogalactoside may be added to enhance the promoter efficiency. When a recombinant microorganism containing an extracellular secretion type plasmid is cultured, culturing is preferably continued in a same medium from when a maximum growth is reached, i.e., late logarithmic phase, until an Fc region protein accumulates in medium. About 12 to 48 hours will elapse from a time when the maximum growth is reached to a time when production and accumulation of Fc region protein ceases. A microorganism containing a periplasmic transportation type plasmid is cultured in the same manner as described above for the microorganism containing an extracellular secretion type plasmid.

After the culturing, the resulting recombinant microbial cells are collected by conventional means, such as centrifugation or filtration, the collected cells are suspended in an aqueous medium such as a phosphate buffer, the suspension treated, for example, by the osmotic shock method (C. Kato et al., E.J. Appl. Microbiol. Biotechnol., 18, 339, 1983), to separate the

intracellular fraction, periplasmic fraction, and extracellular fraction. The amount of human IgG Fc region protein in each fraction can be determined by an enzyme immunoassay using a commercially available rabbit antiserum containing the anti-human IgG Fc component antibody and an enzyme-labeled anti-rabbit Ig antibody.

The human IgG Fc region protein can be purified from the fraction according to a conventional process used for isolation and purification of a protein. Preferably, affinity column chromatography using anti-human IgG Fc component antibody is used. The purified human IgG Fc region protein is subjected to SDS-polyacrylamide gel electrophoresis for determination of the molecular weight, amino acid composition analysis, and determination of the amino acid sequence at the N-terminus, to confirm the secretion of the desired human IgG Fc region protein from which the signal peptide has been correctly clipped off.

In the present specification, when amino acids, peptides, nucleic acids, etc., are expressed by an abbreviation, such abbreviations are used according to the recommendations of the IUPAC-IUB (Commission on Biological Nonmenclature). The optical isomers of the amino acids, unless otherwise noted, have the L configuration. For example, the following abbreviations are used.

| Cys | cysteine |
|-----|----------|
| Met | methionine |
| Glu | glutamic acid |
| Asp | aspartic acid |
| Lys | lysine |
| Arg | arginine |
| His | histidine |
| Phe | phenylalanine |
| Tyr | tyrosine |
| Trp | tryptophan |
| Pro | proline |

| Asn | asparagine |
| Gln | glutamine |
| Gly | glycine |
| Ala | alanine |
| Val | valine |
| Leu | leucine |
| Ile | isoleucine |
| Ser | serine |
| Thr | threonine |
| DNA | deoxyribonucleic acid |
| A | adenine |
| T | thymine |
| G | guanine |
| C | cytosine |
| EDTA | Ethylenediaminetetraacetic acid |
| SDS | sodium dodecyl sulfate. |

Moreover, unless otherwise noted, the microbially produced human immunoglobulin G Fc region protein includes the monomeric and dimeric form.

The purified Fc region protein in monomeric form can be converted to a dimeric form wherein two monomeric proteins are linked via two disulfide bonds similar to natural immunoglobulin by, for example, a method disclosed by Chance et al. (R.E. Chance et al., Peptides: the Seventh U.S. Peptide Symposium Proceedings, D.H. Rich & E. Gross ed., 721-728, Piece Chemical Co., Rockford, IL., 1981).

A dimeric protein can be converted to a monomeric protein by reducing the dimeric protein under the conventional conditions for the reductive cleavage of a disulfide bond, for example, by adding dithiothreitol or 2-mercaptoethanol. A mixture of a monomeric protein and a dimeric protein can be separated into individual forms by any conventional separation procedure, such as gel filtration technique.

Examples

The present invention will now be further illustrated by, but is by no means limited to, the following examples.

Example 1.  Isolation of human chromosomal DNA

$3 \times 10^8$ cells of human lymphoma cell line ARH77 - were disrupted with a glass rod, and the disrupted cells were treated with protease K (Sigma) in the presence of 2% SDS, added with phenol saturated with an aqueous solution containing 10 mM Tris-HCl (pH 8.0) and 1 mM EDTA, and mixed.  The aqueous phase was separated from the phenol phase by centrifugation to obtain the aqueous phase (phenol extraction), which was then dialysed against an aqueous solution containing 20 mM Tris-HCl (pH 7.5), 100 mM NaCl, and 5 mM EDTA.  The dialysate was treated with ribonuclease A (Sigma), again extracted with phenol, and dialysed against an aqueous solution containing 10 mM Tris-HCl (pH 8.0) and 1 mM EDTA to obtain about 1.2 mg of human chromosomal DNA (N. Blin et al., Nucleic Acids Res. 3, 2303, 1976).

Example 2.  Preparation of human gene library

150 µg of the human chromosomal DNA obtained in Example 1 was partially digested with a restriction enzyme Eco RI (Takara Shuzo, Japan), and the digest was subjected to sucrose density gradient centrifugation in 10 to 40% (w/v) sucrose at 28,000 rpm (120,000 xg) for 15 hours at a temperature of 20°C, to obtain 4.3 µg of DNA fragments corresponding to 15 to 23 Kbp.  0.8 µg of the DNA fragments were then ligated with Charon 4A vector to obtain a hybrid DNA comprising the DNA fragments of human origin flanked with the right arm and the left arm of the Charon 4A vector.  The ligation was carried out with T4 DNA ligase (Bethesda Research Laboratories) in an aqueous solution containing 66 mM Tris-HCl (pH 7.6), 6.6 mM $MgCl_2$ , 10 mM dithiothreitol and 1 mM ATP, at 11°C for 12 hours.  The hybrid DNA thus obtained was subjected to in vitro packaging to obtain a human gene library ($1.8 \times 10^6$ PFU/µg, containing 99% of the human chromosomal DNA).

## Example 3.  Screening of human immunoglobulin G gene

The gene library prepared in Example 2, comprising a population of the Charon 4A phage containing the DNA fragment of human origin, was used to transfect E. coli LE 392 to form plaques.  Clones containing the human immunoglobulin gene were selected by a plaque hybridization method using $^{32}$P-labeled human antibody H chain J region gene as a probe.  DNA containing the human immunoglobulin gene was prepared from the Charon 4A phage according to a method of Thomas et al. J. Mol. Biol. 91, 315, 1974.

## Example 4.  Preparation of restriction enzyme cleavage map

1 µg of Charon 4A DNA containing the human immunoglobulin gene was dissolved in 20 µl of a restriction enzyme buffer, the solution was added with 2 to 4 units of a restriction enzyme, and incubated for more than one hour.  The incubation was carried out at 60°C for restriction enzymes Bst NI and Taq I, and at 37°C for other restriction enzymes.  The restriction enzyme buffer was an aqueous solution containing 50 mM Tris-HCl (pH 7.4), 100 mM NaCl, and 10 mM $MgSO_4$ for Eco RI, Taq I, Xba I, and Xho I; an aqueous solution containing 10 mM Tris-HCl (pH 7.5), 60 mM NaCl, and 7 mM $MgCl_2$ for Bam HI, Cla I, Hind III, Pst I, Rsa I, and Sau 3AI; an aqueous solution containing 10 mM Tris-HCl (pH 7.4), 10 mM $MgSO_4$ , and 1 mM dithiothreitol for Bal I, Bst NI, Nae I, and Sst II; and an aqueous solution containing 10 mM Tris-HCl (pH 8.0), 20 mM KCl, 7 mM $MgCl_2$ , and 7 mM 2-mercaptoethanol for Sma I.  Restriction enzymes Bst NI, Cla I and Nae I were obtained from New England Biolabs; Sst II was obtained from Bethesda Research Laboratories; Rsa I, Sau 3AI and Taq I were obtained from Nippon Gene; and other restriction enzymes were obtained from Takara Shuzo.  Where two restriction enzymes were used, DNA was treated first with a restriction enzyme which acts at a lower salt concentration, and after the salt concentration in the reaction mixture was increased, treated with another restriction enzyme which acts at a higher salt concentration.

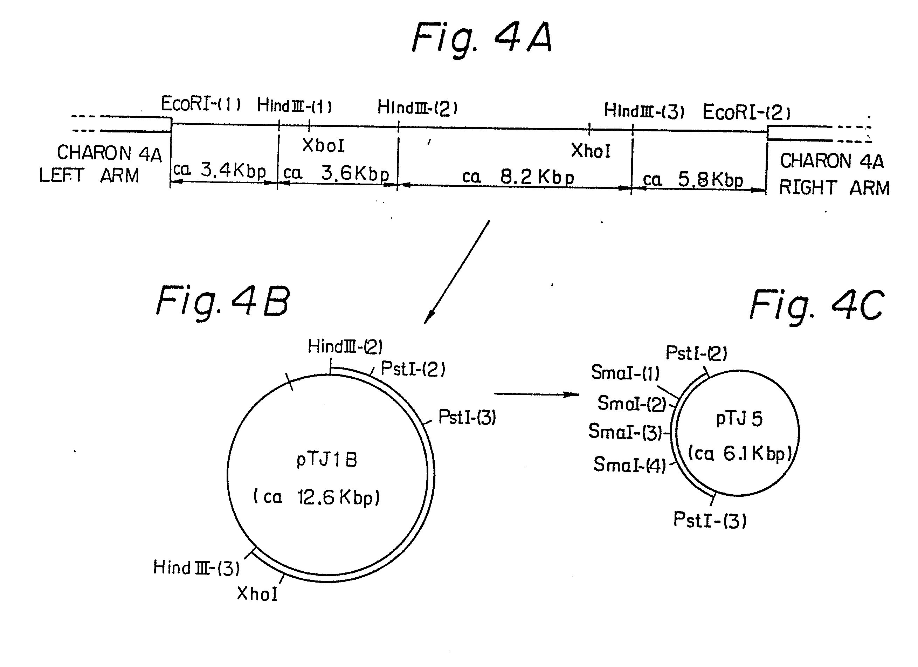

After the cleavage of DNA with the restriction enzyme, the reaction mixture was added with 4 μl of an aqueous solution of 0.25% bromophenol blue in 50% glycerol, and the mixture was subjected to agarose gel electrophoresis, wherein 0.8 to 2.5% of gel concentration was used. This agarose was Type II for electrophoresis use obtained from Sigma. The electrophoresis buffer was an aqueous solution containing 40 mM Tris-$CH_3COOH$ (pH 8.0) and 1 mM EDTA. Electrophoresis was carried out using a vertical gel 2 mm thick, at a voltage of 6 to 9 V/cm for 1.5 to 3 hours. As a molecular weight standard, a cleavage product of λ phage DNA with Hind III (Boehringer Mannhein) was used. After the electrophoresis, DNA in the agarose gel was stained with 2 μg/ml of an ethidium bromide aqueous solution, and the agarose gel was irradiated with long wavelength UV to observe the restriction enzyme cleavage pattern of the DNA. By analyzing cleavage patterns obtained by each restriction enzyme digestion and by combinations of digestion with two restriction enzymes, the relative positions of restriction enzyme cleavage sites for some restriction enzymes were determined as shown in Fig. 4(A). Figure 4(A) represents a restriction map for human chromosomal DNA containing the human immunoglobulin G gene.

Example 5.   Subcloning of human immunoglobulin G
                        gene

3 μg of Charon 4A DNA containing the human immunoglobulin G gene was cleaved with restriction enzyme Hind III according to the same procedure as used in Example 4, and the cleavage product was subjected to agarose gel electrophoresis (gel concentration 0.8%). A gel fragment containing a band corresponding to DNA of about 8.2 Kbp containing the human IgG Fc region gene was excised from the gel, and the excised gel fragment was dissolved in 3 volume/weight of an 8 M $NaClO_4$ aqueous solution. A DNA fragment of about 8.2 Kbp in-length was recovered from the agarose gel solution by a method disclosed by C.W. Chen et al., Anal. Biochem. 101, 339 (1980).

On the other hand, 1 µg of plasmid pBR322 was cleaved with restriction enzyme Hind III according to the procedure described in Example 4, and the reaction mixture was added with 0.5 unit of alkaline phosphatase (E. coli C 75; Takara Shuzo), and reacted at 45°C for one hour. After the reaction, the reaction mixture was extracted three times with phenol to inactivate and eliminate the alkaline phosphatase. This reaction mixture containing pBR322 DNA treated with Hind III and alkaline phosphatase was mixed with the above-described Hind III fragment of 8.2 Kbp recovered from the agarose gel, the mixture then subjected to ethanol precipitation, and the precipitate dissolved in 50 µl of a ligation mixture (see Example 2). The solution was added with 2 units of T4 DNA ligase, and reacted for 12 hours at 11°C to ligate the DNA fragments.

The ligation mixture was used to transform E. coli C 600 r⁻m⁻ (ATCC 33525) by a modified method of a conventional $CaCl_2$ method (M.V. Norgard). That is, E. coli C 600 r⁻m⁻ was cultured for 18 hours to prepare an inoculum, which was then inoculated to 5 ml of L medium (1% trypton, 0.5% of yeast extract, and 0.5% of NaCl, pH 7.2), and the E. coli was cultured to an extent corresponding to an optical density at 600 nm ($OD_{600}$) of 0.3. The cultured cells were washed two times in a cold magnesium buffer (0.1 M NaCl, 5 mM $MgCl_2$, 5 mM Tris-HCl, pH 7.6 at 0°C), resuspended in a cold calcium buffer (100 mM $CaCl_2$, 250 mM KCl, 5 mM $MgCl_2$, 5 mM Tris-HCl, pH 7.6 at 0°C), and the suspension was allowed to stand for 25 minutes at 0°C. The cells were separated by centrifugation and resuspended to a 1/10 volume of the calcium buffer to increase the cell concentration. The concentrated cell suspension was then mixed with the above-mentioned ligated DNA aqueous solution at a volume ratio of 2:1. The mixture was maintained for 60 minutes at 0°C, and added with 1 ml of an LBG medium (1% trypton, 0.5% yeast extract, 1% NaCl and 0.08% glucose, pH 7.2), and culturing was carried out at 37°C for one hour with shaking. 100 μl/plate of the cultured broth was inoculated on a selective medium (L medium plate containing 30 μg/ml of ampicillin), and the plates were incubated at 37°C overnight to develop colonies of transformants. From the ampicillin resistant colonies, DNA was prepared by a conventional method, and analyzed by agarose gel electrophoresis to confirm a desired subclone pTJ1B (about 12.6 Kbp).

A restriction map of the subclone was prepared according to the procedure described in Example 4, and is shown in Fig. 4(B). As shown in Fig. 4(B), it was confirmed that three or four Pst I sites existed between Pst I(3) and Hind III(3). Note, a position thereof was not confirmed.

Moreover, a Pst I(2) - Pst I(3); DNA fragment having a length of 1.7 Kbp was excised and inserted into a Pst I site of plasmid pBR322 to construct a plasmid pTJ5 (about 6.1 Kbp), according to the procedure as described for pTJ1B. A desired clone was selected from the tetracycline resistant transformant, and a restriction map of the subclone thus obtained was as shown in Fig. 4(C).

Example 6.  Sequencing of DNA

A nucleotide sequence of the human immunoglobulin G Fc region gene was determined according to the Maxam-Gilbert method.

For example, about 50 μg of DNA of the subclone pTJ5 constructed as shown in Example 5 was cleaved with Sma I according to the procedure described in Example 4, the resulting DNA fragments were dephosphorylated with alkaline phosphatase, and labeled with $[\gamma\text{-}^{32}P]$-ATP using 5 units of polynucleotidekinase (P-L Biochemicals). The reaction using polynucleotidekinase was carried out in an aqueous solution containing 50 mM Tris-HCl (pH 9.5), 10 mM $MgCl_2$, and 5 mM dithiothreitol with 100 µCi of $[\gamma\text{-}^{32}P]$-ATP (Amersham). The $^{32}P$-labeled DNA fragments were cleaved with Pst I, desired DNA fragments were separated by polyacrylamide gel electrophoresis (gel concentration 5%), and extracted from the gel according to the procedure described hereinafter in Example 7. The $^{32}P$-labeled Sma I - Pst I fragments thus obtained were subjected to a base-specific partial cleavage, and the product was separated by polyacrylamide gel electrophoresis (gel concentration 8 to 23%) in 7 M urea. The gel was subjected to autoradiography at -80°C for 1 day, the cleavage pattern was analyzed, and the result was used to determine the nucleotide sequence of the Fc region gene.

On the other hand, DNA of plasmid PTJ5 was cleaved with Pst I, and the resulting DNA fragment was labeled with $[\alpha\text{-}^{32}P]$-ddATP using a 3'-terminal labeling kit (Amersham). The $^{32}P$-labeled DNA fragments were cleaved with Sma I, and desired DNA fragments were separated and purified by polyacrylamide gel electrophoresis (gel concentration 5%). The $^{32}P$-labeled Pst I - Sma I fragments were analyzed as described above, and the result was used to determine a nucleotide sequence of the Fc region gene.

Example 7. Construction of expression plasmid
(cloning of $C_H3$ domain gene)

DNA of plasmid pTJ5 constructed in Example 5 was cleaved with restriction enzyme Pst I according to the procedure described in Example 4, the resulting DNA fragments were separated by agarose gel electrophoresis (gel concentration 0.8%), and a DNA fragment of about 1.7 Kbp containing the Fc region gene was recovered from the gel by the procedure described in Example 5. The DNA fragment thus obtained was cleaved with restriction enzyme Nae I by the procedure described in Example 4, and the resulting DNA fragments were separated by polyacrylamide gel electrophoresis (gel concentration 5%). A gel fragment containing a band corresponding to a DNA fragment of about 0.6 Kbp containing a $C_H3$ domain gene was excised and disrupted, the disrupted gel particles were added with 2 to 5 ml of an elution buffer (500 mM ammonium acetate, 1 mM EDTA and 0.1% SDS, pH 7.5), and the mixture was shaken at 37°C overnight. The mixture was centrifuged to recover an aqueous phase containing the desired DNA fragment. The DNA fragment thus obtained was then cleaved with restriction enzyme Rsa I by the procedure described in Example 4, by polyacrylamide gel electrophoresis (gel concentration 5%), and a DNA fragment of about 310 bp containing a $C_H3$ domain gene was recovered from the gel as above mentioned.

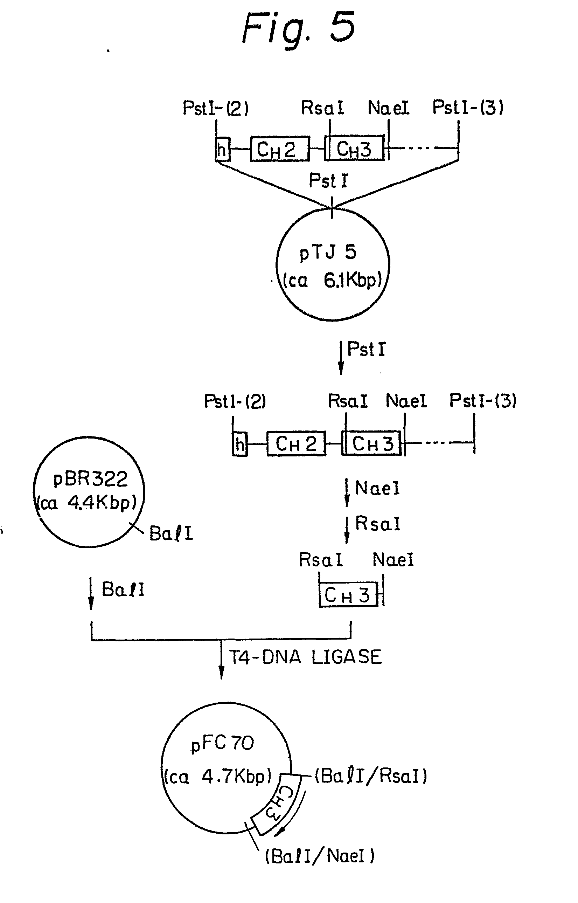

The Rsa I - Nae I DNA fragment containing the $C_H3$ domain gene was inserted into the Bal I site of the plasmid pBR322 according to a procedure similar to the procedure described in Example 5, to construct a plasmid pFC70 (about 4.7 Kbp) wherein the reading orientation of the $C_H3$ domain gene was the same as the reading orientation of the tetracycline resistance gene in pBB322, i.e., clockwise orientation in Fig. 5). The process for constructing the pFC70 is shown in Fig. 5.

<u>Example 8.</u>  <u>Construction of expression plasmid</u>
<u>(ligation of $C_H2$ domain gene and</u>
<u>$C_H3$ domain gene)</u>

The DNA fragment of 1.7 Kbp containing the Fc region gene obtained in Example 7 was cleaved with restriction enzymes Sau 3AI and Taq I, the resulting DNA fragments were separated by polyacrylamide gel electrophoresis (gel concentration 5%), and about 0.5 µg of DNA

fragment having a length of about 240 bp containing the $C_H2$ domain gene was recovered from the gel by the procedure described in Example 7.

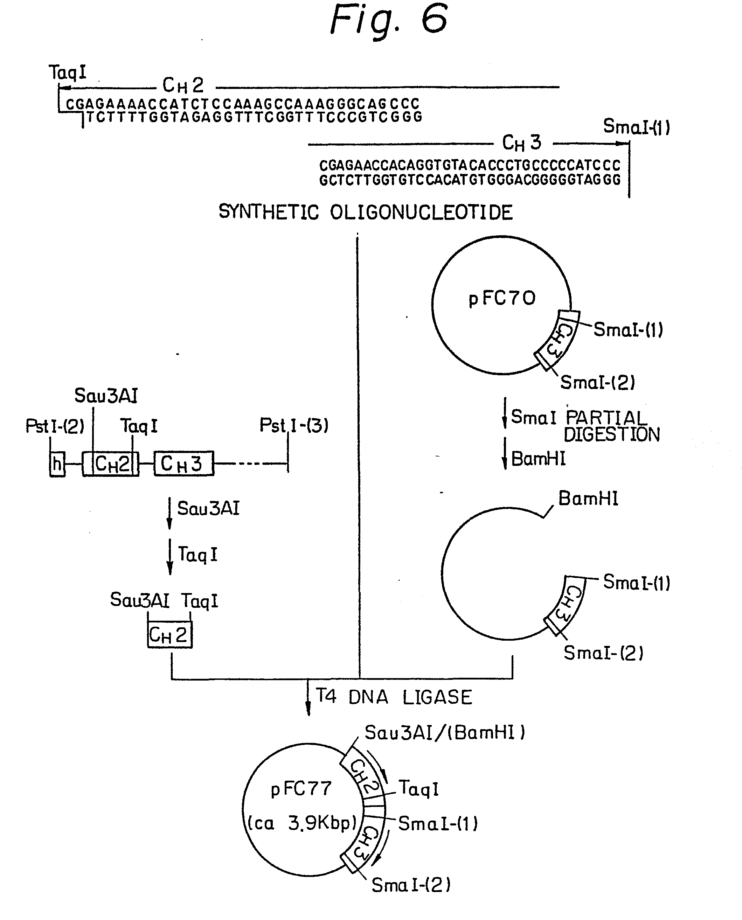

A double stranded oligonucleotide corresponding to a joint region between the $C_H2$ domain gene and $C_H3$ domain gene, having a nucleotide sequence shown in Fig. 6, was prepared by separately synthesizing a single stranded coding chain and a non-coding chain.

The single stranded oligonucleotides were synthesized by the phophoramidite method using a fully-automatic DNA synthesizer (Applied Biosystems, Model 380A). The synthetic oligonucleotides were purified according to a manual disclosed by Applied Biosystems. That is, an aqueous ammonium solution containing synthetic oligonucleotides was maintained at 55°C overnight to deprotect the DNA base, and the solution was subjected to gel filtration using Sephadex G-50 fine gel (Pharmacia) to isolate a synthetic oligonucleotide with a high molecular weight. Next, the isolated product was subjected to polyacrylamide gel electrophoresis (gel concentration 20%) in 7 M urea, and a migration pattern of the gel was observed to determine the position of a band corresponding to a desired oligonucleotide size. The desired part of the gel thus determined was then excised, and a desired synthetic oligonucleotide was recovered from the excised gel piece according to the procedure described in Example 7. Finally, the oligonucleotide solution was applied on a gel filtration column (Sephadex G-50) to obtain a purified synthetic oligonucleotide preparation. If necessary, the polyacrylamide gel electrophoresis was repeated to improve the purify of the oligonucleotide product. 0.1 to 1.0 μg of the purified synthetic oligonucleotide was treated with polynucleotide kinase in the presence of 1 mM ATP as described in Example 6, to phosphorylate the 5'-end of the oligonucleotide. Two phosphorylate synthetic oligonucleotides corresponding to the coding chain and non-coding chain were mixed, and the mixture was gradually cooled from 70°C to room temperature for annealing.  As described above, a Taq I - Sma I DNA fragment of about 68 bp corresponding to a joint region between the $C_H2$ domain gene and $C_H3$ domain gene was obtained.

On the other hand, about 5 μg of DNA of the plasmid pFC70 was dissolved in the cleavage buffer for restriction enzyme Sma I as described in Example 4, the solution was added with 2 to 5 units of Sma I, and incubated at 20°C for 15 to 45 minutes for partial digestion.  The reaction mixture was extracted with phenol to inactivate the Sma I, and subjected to cleavage with Bam HI according to the procedure described in Example 4.  The resulting DNA fragments were separated by agarose gel electrophoresis (gel concentration 0.8%), and a Bam HI - Sma I(1) DNA fragment of about 3.6 Kbp containing most of the $C_H3$ domain gene, as shown in Fig. 6, was recovered from the gel by the procedure described in Example 5.

The Sau 3AI - Taq I DNA fragment containing the $C_H2$ domain gene; the Taq I - Sma I DNA fragment corresponding to the joint region between the $C_H2$ domain gene and $C_H3$ domain gene; and the Bam HI (Sau 3AI) - Sma I(1) DNA fragment containing the $C_H3$ domain gene and a vector fragment, all prepared as above-mentioned, were mixed and ligated according to a procedure roughly the same as the procedure described in Example 5, to construct a plasmid pFC77 of about 3.9 Kbp wherein the $C_H2$ domain gene and the $C_H3$ domain gene were linked without an intervening intron, i.e., directly.  The above-mentioned process for constructing the plasmid pFC77 is shown in Fig. 6.

Example 9.  Ligation of Fc region gene and promoter

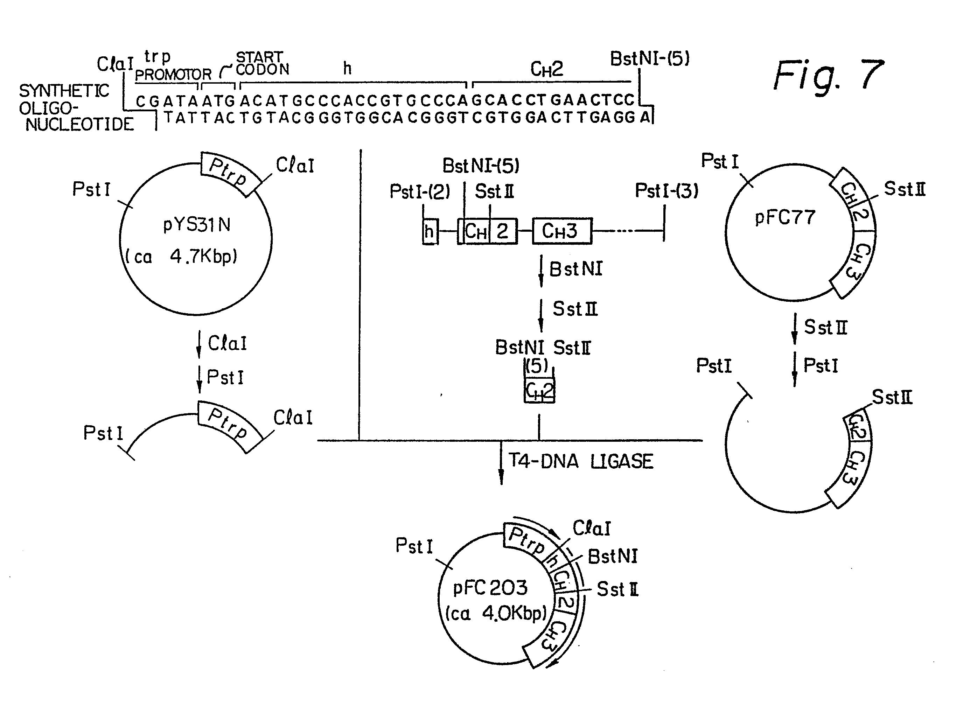

The DNA of the plasmid pFC77 obtained in Example 8 was cleaved with restriction enzymes Sst II and Pst I by the same procedure described in Example 4, and after agarose gel electrophoresis (gel concentration 0.8%), an Sst II - Pst I DNA fragment of about 2.7 Kbp containing a downstream part of the $C_H2$ domain gene, the entire $C_H3$ domain gene, and a portion of the vector region, as shown in Fig. 7, was recovered from the gel by the procedure described in Example 5.

On the other hand, the DNA fragment of about 1.7 Kbp containing the Fc region gene obtained in Example 7 was cleaved with restriction enzymes Bst NI and Sst II by the procedure described in Example 4, and after polyacrylamide gel electrophoresis (gel concentration 5%), a Bst NI(5) - Sst II DNA fragment of about 171 bp containing an upstream part of the $C_H2$ domain gene was recovered from the gel by the procedure described in Example 7.

Moreover, a double stranded oligonucleotide of about 39 bp corresponding to the link region between a promoter and Fc region gene, shown in Fig. 7, was prepared by the procedure described in Example 8. This Cla I - Bst NI(5) DNA fragment contains a restriction enzyme Cla I site for ligation to the trp promoter, a translation start codon ATG, an h domain gene, and an upstream part of the $C_H2$ domain gene, in sequence in this order. Therefore, by using this DNA fragment, the Fc region (h-$C_H2$-$C_H3$) gene without any intervening introns can be linked downstream of the tryptophan·operon·SD sequence within an appropriate distance.

On the other hand, the DNA of the plasmid pYS31N of about 4.7 Kbp containing the trp promoter was cleaved with restriction enzymes Pst I and Cla I by the procedure described in Example 4, and after agarose gel electrophoresis (gel concentration 0.8%), a Pst I - Cla I DNA fragment of about 1.1 Kbp containing the trp promoter and a part of the vector region, as shown in Fig. 7, was recovered from the agarose gel by the procedure described in Example 5.

The Sst II - Pst I DNA fragment containing a downstream part of the $C_H2$ domain gene, $C_H3$ domain gene, and a portion of the vector region; a Bst NI(5) - Sst II DNA fragment containing an upstream part of the $C_H2$ - domain gene; the Cla I - Bst NI(5) DNA fragment corresponding to the link region between the promoter and the Fc region gene; and the Pst I - Cla I DNA fragment containing the trp promoter and a part of the vector region, all prepared as described above, were mixed and ligated to construct an expression plasmid pFC203 of about 4.0 Kbp for the Fc region (h-$C_H2$-$C_H3$) gene by a procedure roughly the same as the procedure described in Example 5. The above-mentioned process for construction of the pFC203 is shown in Fig. 7.

Example 10. Incorporation of tandem stop codons

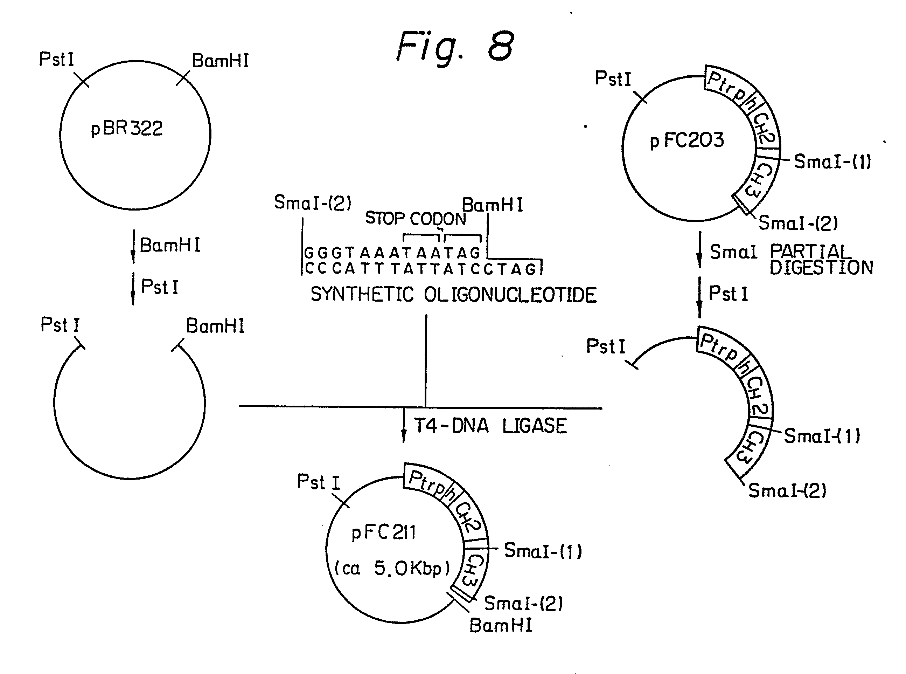

DNA of the Fc region gene expression plasmid pFC203 constructed in Example 9 was partially cleaved with restriction enzyme Sma I, and then completely cleaved with restriction enzyme Pst I by        the same procedure as described in Example 8. After agarose gel electrophoresis (gel concentration 0.8%), a Sma I(2) - Pst I DNA fragment of about 1.8 Kbp containing a major portion of the Fc region gene and a portion of the vector region, as shown in Fig. 8, was recovered from the agarose gel by the same procedure described in Example 5.

On the other hand, a double stranded oligonucleotide of about 17 bp having the nucleotide sequence shown in Fig. 8 corresponding to a downstream part of the $C_H3$ domain gene and translation stop codons was prepared by the procedure described in Example 8. This Sma I(2) - Bam HI DNA fragment contained a portion of the $C_H3$ domain gene, two tandem translation stop codons represented by the nucleotide sequence TAATAG, and a restriction enzyme Bam HI site for ligation to a vector. Therefore, this DNA fragment can be used to incorporate tandem translation stop codons into an expression plasmid.

The plasmid pBR322 DNA was cleaved with restriction enzymes Pst I and Bam HI by the procedure described in Example 4, and after agarose gel electrophoresis (gel-concentration 0.8%), a Bam HI - Pst I DNA fragment of about 3.2 Kbp containing a major portion of the vector region, as shown in Fig. 8, was recovered from the agarose gel by the procedure described in Example 5.

The Sma I(2) - Pst I DNA fragment containing a major portion of the Fc region gene and a portion of the vector region; the Sma I(2) - Bam HI DNA fragment containing a downstream part of the $C_H3$ domain gene and tandem translation stop codons; and the Bam HI - Pst I DNA fragment containing a major portion of the vector region, all prepared as described above, were mixed and ligated to construct the Fc region gene expression plasmid pFC211 of about 5.0 Kbp having tandem translation stop codons, by        the same procedure as described in Example 5.  The above-mentioned process for the construction of the pFC211 is shown in Fig. 8.

Example 11.  Linking of Fc region gene with tac promoter

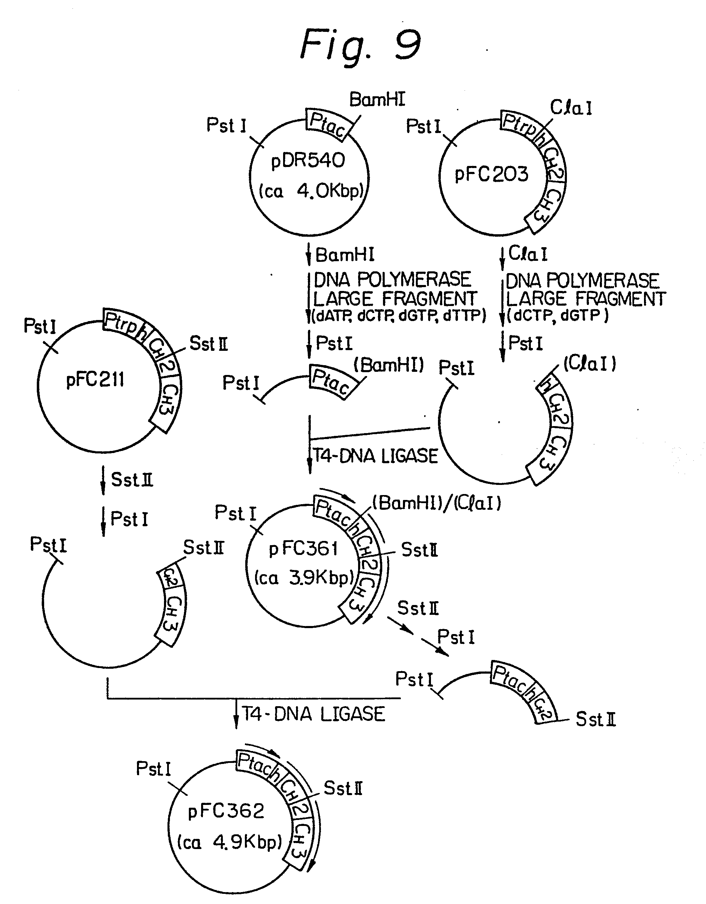

DNA of the Fc region gene expression plasmid pFC203 obtained in Example 9 was cleaved with restriction enzyme Cla I by the procedure described in Example 4, and the resulting DNA was treated with DNA polymerase I large fragment in the presence of dCTP and dGTP to produce blunt ends by the procedure described in Example 10.  Next, the blunt-ended DNA fragment was cleaved with restriction enzyme Pst I by the procedure described in Example 4, and after agarose gel electrophoresis (gel concentration 0.8%), a DNA fragment of about 2.8 Kbp containing an entire Fc region gene and a major portion of the vector region, as shown in Fig. 9, was recovered.

On the other hand, DNA of the plasmid pDR540 of about 4.0 Kbp (Pharmacia) containing the tac promoter was cleaved with restriction enzyme Bam HI by the procedure described in Example 4, and the resulting DNA was treated with DNA polymerase I large fragment in the presence of dGTP, dATP, dTTP, and dCTP to produce blunt ends, by the procedure described in Example 10. The blunt ended DNA fragment was then cleaved with restriction enzyme Pst I by the procedure described in Example 4, and after agarose gel electrophoresis (gel concentration 0.8%), a DNA fragment of about 1.1 Kbp containing the tac promoter and a portion of the vector region, as shown in Fig. 9, was recovered from the agarose gel.

The DNA fragment of about 2.8 Kbp containing the entire Fc region gene and a major portion of the vector region, and the DNA fragment of about 1.1 Kbp containing the tac promoter and a portion of the vector region, both prepared as above, were mixed and ligated to construct an expression plasmid pFC361 of about 3.9 Kbp, wherein the Fc region gene was linked downstream from the tac promoter, by roughly the same method as described in Example 5. Figure 9 shows the above-mentioned process for the construction of the pFC361.

The DNA of the Fc region gene expression plasmid pFC361 thus obtained was cleaved with restriction enzymes Sst II and Pst I by the procedure described in Example 4, and after agarose gel electrophoresis (gel concentration 0.8%), an Sst II - Pst I DNA fragment of about 1.3 Kbp containing a portion of the vector region, the tac promoter, and an upstream portion of the Fc region gene, as shown in Fig. 9, was recovered from the agarose gel by the procedure described in Example 5.

On the other hand, the DNA of the Fc region gene expression plasmid pFC211 having tandem translation stop codons, obtained in Example 11, was cleaved with restriction enzymes Sst II and Pst I by the procedure described in Example 4, and then a Pst I - Sst II DNA fragment of about 3.6 Kbp containing a downstream part of the Fc region gene, tandem translation stop codons, and a major portion of the vector region, as shown in Fig. 9, was obtained as described above.

The Sst II - Pst I DNA fragment containing a portion of the vector region, the tac promoter, and an upstream portion of the Fc region gene; and the Pst I - Sst II DNA fragment containing a downstream portion of the Fc region gene, tandem translation stop codons, and a major portion of the vector region, both prepared as above, were mixed and ligated to construct an expression plasmid pFC362 of about 4.9 Kbp wherein the tac promoter was followed by the Fc region gene with tandem translation stop codons, by roughly the same procedure as described in Example 5. Figure 9 shows the above-mentioned process for the construction of the pFC362.

Example 12. Preparation of chromosomal DNA of alkalophilic Bacillus No. 170

Alkalophilic Bacillus No. 170 (FERM BP-467) capable of extracellular secretion of penicillase was cultured in a medium containing 2.0 g/l glycerol, 5.0 g/l yeast extract, 5.0 g/l polypepton (Takeda), 1 g/l $K_2HPO_4$ and 0.2 g/l $MgSO_4 \cdot 7H_2O$ (pH 9.0 with 10 g/l $NaHCO_3$) at 30°C for 15 hours with shaking, until the late logarithmic phase was reached, and the cultured cells were collected. Chromosomal DNA was extracted from the cells according to the phenol extraction method to obtain 5 mg of chromosomal DNA.

Example 13. Insertion of chromosomal DNA fragment derived from alkalophilic Bacillus No. 170 into vector

10 µg of chromosomal DNA extracted in Example 12 was partially digested with restriction enzyme Eco RI at 37°C according to the same procedure described in Example 4. On the other hand, DNA of plasmid pMB9 carrying a tetracycline resistance gene (Tet[r]) (Bethesda Research Laboratories) was completely digested with the restriction enzyme Eco RI, and the digestion product was heated at 65°C for five minutes, and mixed with the digestion product of chromosomal DNA as above prepared. The mixture was subjected to ligation using T4 DNA ligase at 10°C for 24 hours according to the procedure described in Example 2, and after heating at 65°C for 5 minutes, two volumes of ethanol was added to the reaction mixture to precipitate plasmid DNA.

Example 14.  Cloning of penicillinase gene derived from alkalophilic Bacillus No. 170

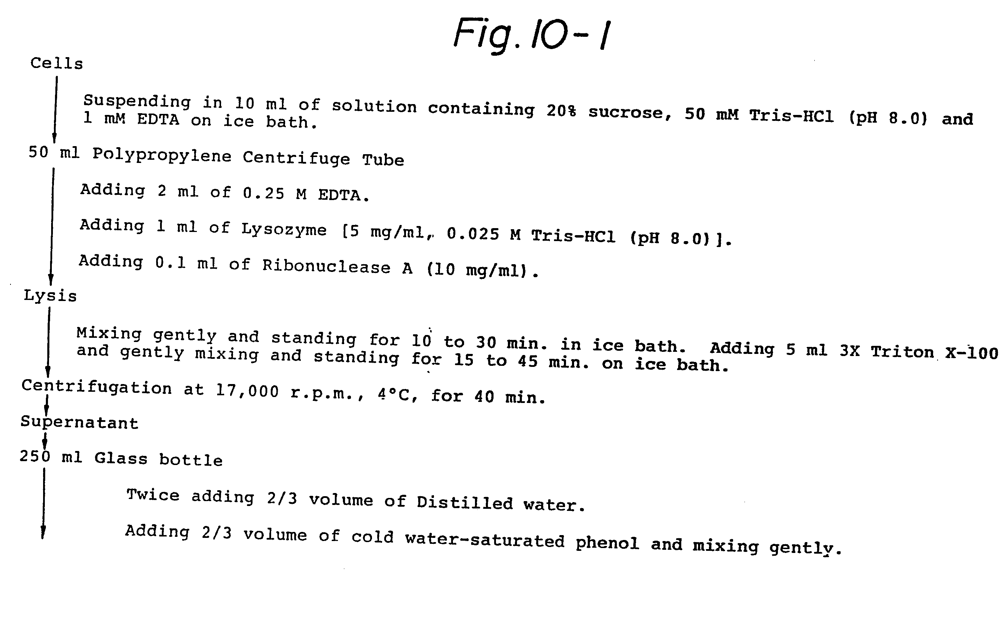

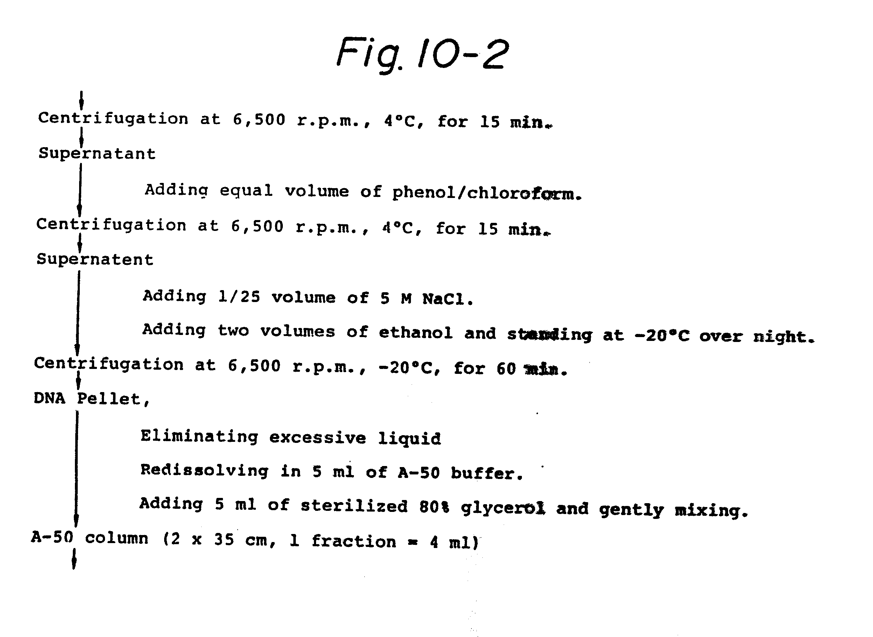

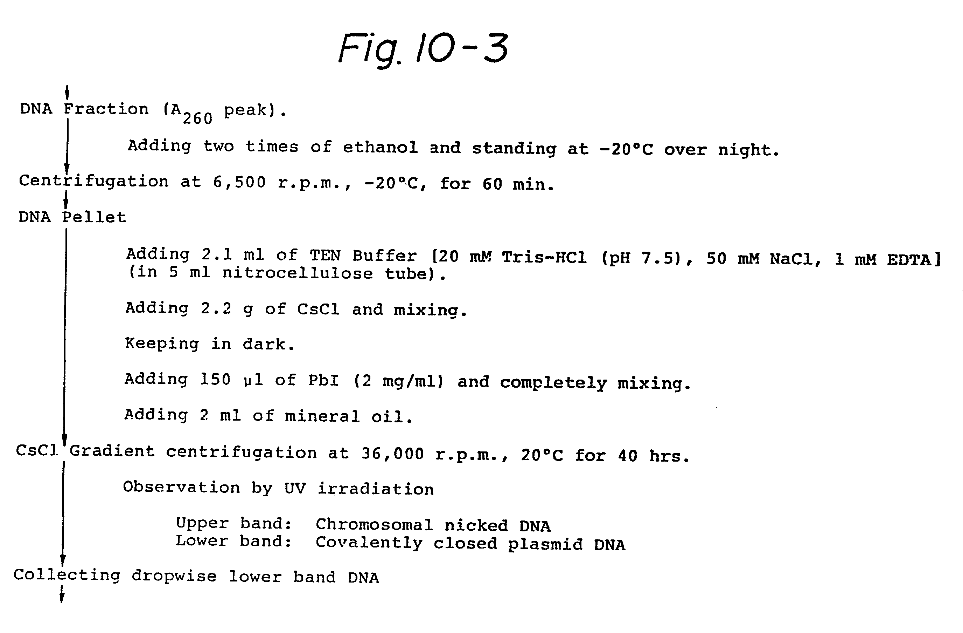

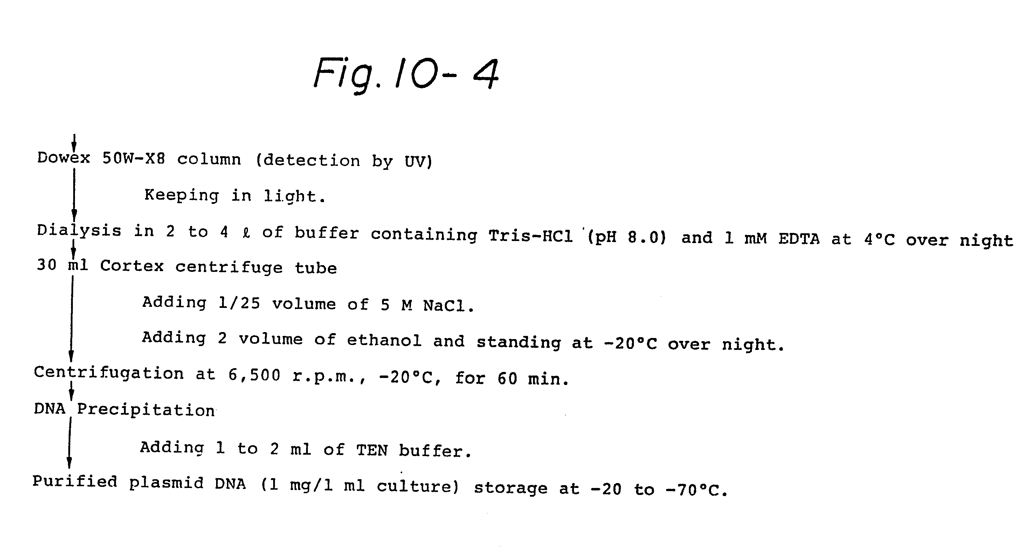

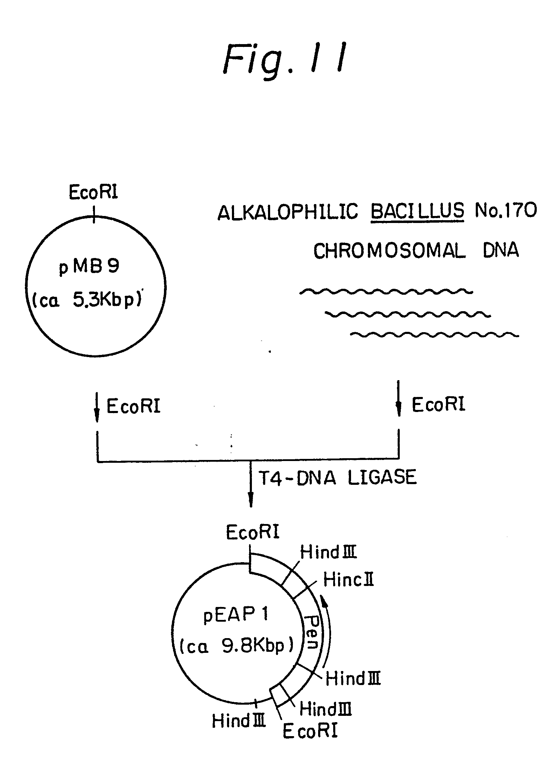

Plasmid containing a chromosomal DNA insert derived from alkalophilic Bacillus No. 170, prepared in Example 13, was transformed into E. coli HB101 by the conventional CaCl$_2$ method (M.V. Norgard et al., supra). The transformants were screened for ampicillin resistance and tetracycline resistance, and a clone containing a penicilinase gene of alkalophilic Bacillus No. 170 was selected.  After culturing the selected strain, the cultured cells were treated according to the procedure described in Fig. 10 to isolate a recombinant plasmid pEAP1 containing a 4.5 Kbp insert comprising the penicillinase gene.  A restriction cleavage map for the plasmid pEAP1 was prepared according the same procedure as described in Example 4.  Figure 11 represents the restriction cleavage map of the plasmid pEAP1.

Example 15.  Determination of nucleotide sequence of plasmid containing chromosomal DNA of alkalophilic Bacillus No. 170

The nucleotide sequence of plasmid containing chromosomal DNA of alkalophilic Bacillus No. 170 was determined according to a dideoxy chain termination method by M13 phage using an M13 Sequencing kit (Amersham).  Figure 1 represents the DNA sequence of the promoter region and signal peptide region of the penicillinase gene of alkalophilic Bacillus No. 170; Fig. 2 represents the DNA sequence of Kil gene derived from plasmid pMB9; and Fig. 3 represents the DNA sequence of the Ex promoter region derived from chromosomal DNA

of alkalophilic <u>Bacillus</u> No. 170.

Example 16. <u>Construction of various plasmid</u>
<u>derivatives containing chromosomal DNA</u>
<u>of alkalophilic Bacillus No. 170</u>  -

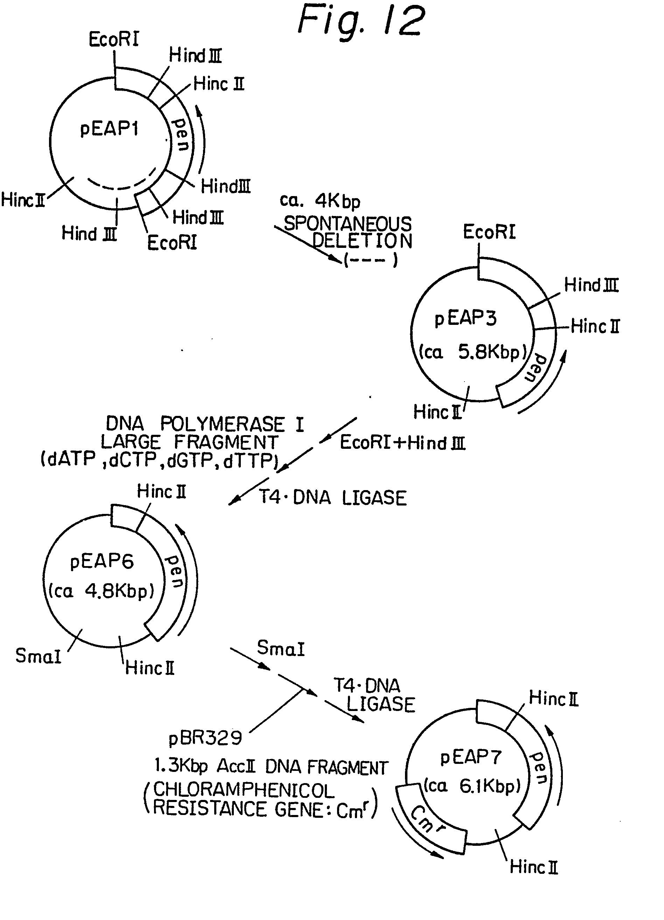

During the passage of <u>E. coli</u> containing the plasmid pEAP1, a mutant (ampicillin resistant, Ap$^r$; tetracycline sensitive, Tet$^s$) exhibited an enhanced penicillinase activity three times higher than that of pEAP1 was obtained spontaneously. From the mutant, a plasmid was prepared according to the procedure described in Fig. 10, and plasmid pEAP3 of about 5.8 Kbp, wherein a DNA fragment of about 4 Kbp corresponding to a DNA region upstream from the penicillinase structural gene in plasmid pEAP1 has been deleted, was obtained. Figure 12 represents the restriction - cleavage map of plasmid pEAP3.

One µg of the plasmid pEAP3 thus obtained was digested with the restriction enzyme Eco RI and Hind III at 37°C for 2 hours according to the same procedure as described in Example 4. To the reaction mixture was added a DNA polymerase I large fragment, and reaction was carried out at a room temperature for 30 minutes to make blunt ends, according to the same procedure as described in Example 11. Next, the ligation of DNA fragments was carried out using T4 DNA ligase at a room temperature for 24 hours, and then the reaction mixture was heated at 65°C for 5 minutes, and two volumes of ethanol was added to the reaction to precipitate and obtain plasmid DNA. The plasmid DNA thus obtained was used to transform <u>E. coli</u> HB101 according to the procedure described in Example 4, and from an ampicillin resistant transformant, plasmid pEAP6 was isolated and purified according to the procedure described in Fig. 10. In the plasmid pEAP6 of about 4.8 Kbp, an Eco RI - Hind III DNA fragment of about 1.0 Kbp was deleted from the original plasmid. Figure 12 represents the process for the construction of plasmid pEAP6.

Next, 10 µg of DNA of plasmid pBR329 (about 4.15 Kbp; L. Covarrubias et al., Gene, 17, 79, 1982) was cleaved with restriction enzyme Acc II, and the reaction mixture was subjected to an agarose gel electrophoresis (gel concentration 1.5%), and from the agarose gel, an Acc II - Acc II DNA fragment of about 1.3 Kbp containing the chloramphenicol acetyl transferase (CAT) gene was recovered by an electroelution method (P.J. Green et al., Methods in Molecular Biology, Vol 7, Marcel Dekker, 1978, p87). On the other hand, the plasmid pEAP6 obtained as described above was cleaved with restriction enzyme Sma I according to the same procedure as described in Example 4, and the reaction mixture was mixed with the above-mentioned Acc II - Acc II DNA fragment, and ligation was carried out using T4 DNA ligase according to the same procedure as described in Example 2. As described above, the reaction mixture was used to transform E. coli HB101, and from a chloramphenicol and an ampicillin resistant transformant plasmid pEAP7 of about 6.1 Kbp was isolated and purified. In the plasmid pEAP7, the CAT gene was inserted into plasmid pEAP6 in the counterclockwise direction in Fig. 12. Figure 12 represents the process for the construction of the plasmid pEAP7.

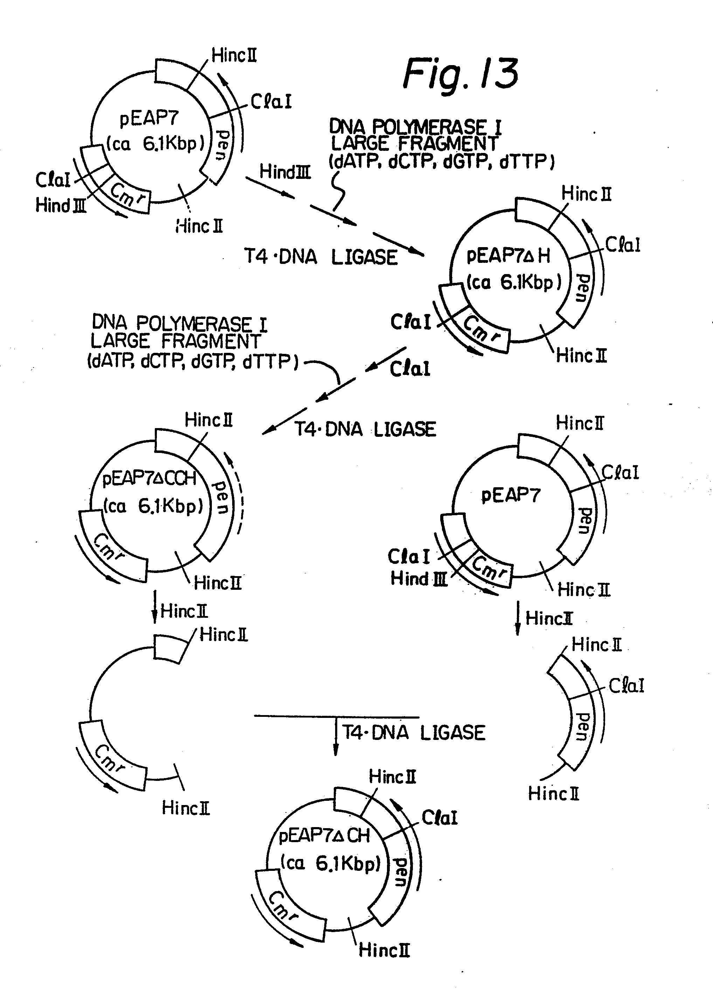

The plasmid pEAP7 thus obtained was cleaved with restriction enzyme Hind III according to the same procedure as described in Example 4, and DNA polymerase I large fragment was used to make blunt ends according to the same procedure as described in Example 11. Self ligation was carried out using T4 DNA ligase according to the same procedure as described in Example 2, to construct a plasmid pEAP7ΔH of about 6.1 Kbp wherein the Hind III site in plasmid pEAP7 has been deleted. Figure 13 represents a process for the construction of the plasmid pEAP7ΔH.

Moreover, plasmid pEAP7ΔH was cleaved with restriction enzyme Cla I according to the same procedure as described in Example 4, and DNA polymerase I large fragment was used to make blunt ends according to the same procedure as described in Example 11. Ligation was carried out using T4 DNA ligase according to the same procedure as described in Example 2, and the reaction mixture was used to transform E. coli HB101. From a chloramphenicol resistant and ampicillin sensitive transformant, plasmid pEAP7ΔCCH of about 6.1 Kbp was isolated and purified. In the plasmid pEAP7ΔCCH, both of the two Cla I sites present in plasmid pEAP7ΔH have been deleted. Figure 13 represents the process for the construction of the plasmid pEAP7ΔCCH.

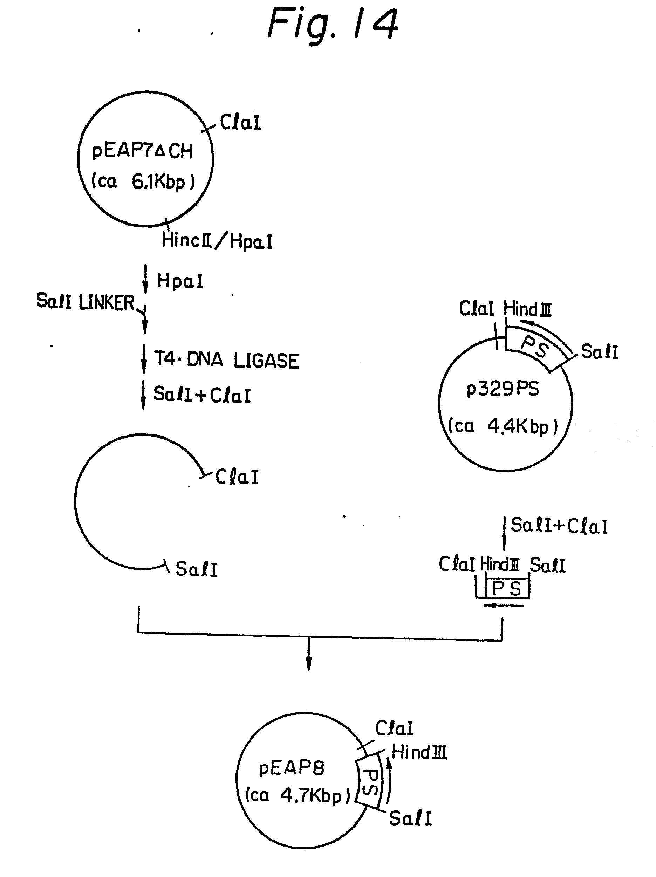

The plasmid pEAP7ΔCCH thus obtained was cleaved with restriction enzyme Hinc II according to the same procedure as described in Example 4, and after an agarose gel electrophoresis (gel concentration 0.8%), an Hinc II - Hinc II DNA fragment of about 3.8 Kbp wherein a portion of the penicillinase gene has been deleted was recovered from the agarose gel by an electroelution method. On the other hand, the above-mentioned plasmid pEAP7 was cleaved with restriction enzyme Hinc II in the same manner as described above, and after an agarose gel electrophoresis (gel concentration 0.8%), an Hinc II - Hinc II DNA fragment of about 2.3 Kbp containing the penicillinase gene was recovered. These two DNA fragments were ligated using T4 DNA ligase according to the same procedure as described in Example 2, and the reaction mixture was used to transform E. coli HB101. From a chloramphenicol and ampicillin resistant transformant, plasmid pEAP7ΔCH of about 6.1 Kbp was obtained. Figure 14 represents the process for construction of the plasmid pEAP7ΔCH.

The plasmid pEAP7ΔCH thus obtained was cleaved with restriction enzyme Hpa I according to the same procedure as described in Example 4, and the reaction mixture was then mixed with a SalI linker phosphorylated at its 5'-ends (Takara Shuzo), and ligation was carried out using T4 DNA ligase according to the same procedure as described in Example 2. After ethanol precipitation, the ligation product was cleaved with restriction enzymes Cla I and Sal I, and after an agarose gel electrophoresis (gel concentration 0.8%), a Cla I - Sal I DNA fragment of about 4.5 Kbp containing the 3'-terminal half of penicillinase gene, Ex·promoter region, and a major vector portion was recovered by an electroelution method. On the other hand, the plasmid p329PS obtained in Example 16 described later was cleaved with restriction enzymes Cla I and Sal I, and after a polyacrylamide gel electrophoresis (gel concentration 5%), a Sal I - Cla I DNA fragment of about 200 bp containing the penicillinase gene promoter region and signal peptide region was recovered by an electroelution method. The two DNA fragments thus obtained were mixed, after ligation using T4 DNA ligase according to the same procedure as described in Example 2, the reaction mixture was used to transform E. coli HB101 in the same manner as described above. From a chloramphenicol resistant transformant, secretion type plasmid, a vector pEAP8 of 4.7 Kbp was isolated and purified. Figure 14 represents the process for the construction of the plasmid pEAP8.

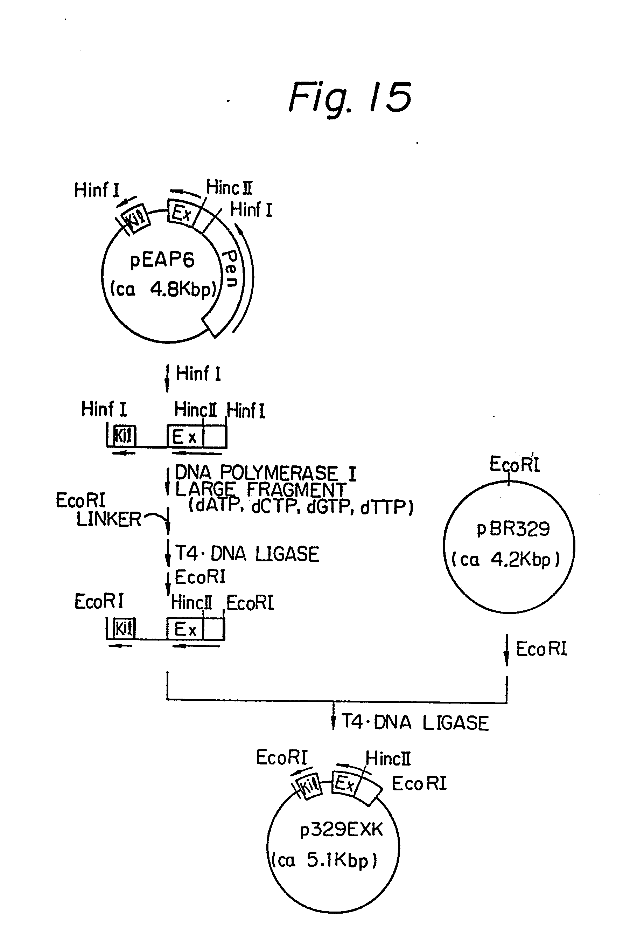

The plasmid pEAP6 prepared as described above was cleaved with restriction enzyme Hinf I according to the same procedure as described in example 4, and after an agarose gel electrophoresis (gel concentration 0.8%), Hinf I - Hinf I DNA fragment containing the Ex promoter region derived from chromosomal DNA of alkalophilic Bacillus No. 170 and the Kil gene region responsible for extracellular secretion derived from plasmid pMB9 was recovered by electroelution. This DNA fragment was treated with DNA polymerase I large fragment to make blunt ends according to the same procedure as described in Example 11, and the fragment was ligated with an 5'-end-phosphorylated Eco RI linker (Takara Shuzo) using T4 DNA ligase according to the same procedure as described in Example 2. After ethanol precipitation, the ligation product was cleaved with restriction enzyme Eco RI according to the same procedure as described in Example 4. Next, after a agarose gel electrophoresis - (gel concentration 0.8%), an Eco RI - Eco RI DNA fragment of about 0.95 Kbp was recovered by an electroelution method. Next, plasmid pBR329 was cleaved with restriction enzyme Eco RI according to the same procedure as described in Example 4, and the reaction product was mixed with the above-mentioned Eco RI - Eco RI DNA fragment of about 0.95 Kbp, and the mixture was subjected to ligation using T4 DNA ligase according to the same procedure as described in Example 2. The ligation product was used to transform E. coli HB101, and from an ampicillin and tetracycline resistant transformant, multi-copy secretion type plasmid, a vector p329EXK of about 5.1 Kbp was isolated and purified. Figure 15 represents the process for the construction of the plasmid p329EXK.

Example 17. Construction of plasmid containing a promoter region and signal peptide region of penicillinase gene of alkalophilic Bacillus No. 170

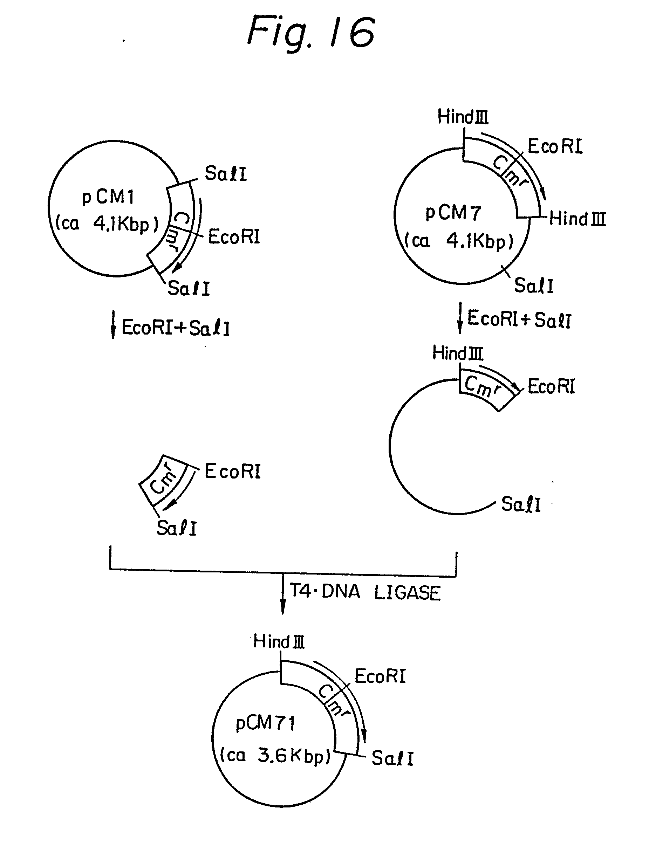

Plasmid pCM1 (about 4.1 Kbp Pharmacia) which contains chloramphenicol acetyl transferase (CAT) gene derivative was cleaved with restriction enzymes Eco RI and Sal I according to the same procedure as described in Example 4, and after an agarose gel electrophoresis (gel concentration 0.8%), an Eco RI - Sal I DNA fragment of about 0.5 Kbp containing the 3'-terminal half of CAT gene was recovered according to the same procedure as described in Example 15. Moreover, plasmid pCM7 (about 4.1 Kbp, Pharmacia) which contains a CAT gene derivative was cleaved with restriction enzymes Eco RI and Sal I as described above, and after an agarose gel electrophoresis (gel concentration 0.8%), an Eco RI - Sal I DNA fragment of about 3.1 Kbp consisting of a 5'-terminal half of the CAT gene and a major vector portion was recovered. These DNA fragments were mixed and ligated using T4 DNA ligase according to the same procedure as described in Example 2, and a new plasmid pCM71 of about 3.6 Kbp containing the CAT gene derivative was constructed according to the same procedure as described in Example 16. Figure 16 represents the process for the construction of the plasmid pCM71.

The plasmid pEAP3 constructed in Example 16 was cleaved with restriction enzyme Rsa I according to the same procedure as described in Example 4, and after polyacrylamide gel electrophoresis (gel concentration 5%), an Rsa I - Rsa I DNA fragment of about 200 bp containing the promoter region and signal peptide region of the penicillinase gene was recovered according to the same procedure as described in Example 16. This Rsa I - Rsa I DNA fragment was mixed with an 5'-end-phosphorylated Hind III linker (Takara Shuzo), and after ligation using T4 DNA ligase according to the same procedure as described in Example 2, the ligation product was cleaved with restriction enzyme Hind III according to the same procedure as described in Example 4. After polyacrylamide gel electrophoresis (gel concentration 5%), a Hind III - ended DNA fragment of about 210 bp containing the promoter region and signal peptide region of the penicillinase gene was recovered according to the same procedure as described in Example 15. On the other hand, plasmid pCM71 was cleaved with restriction enzyme Hind III according to the same procedure as described in Example 4, and the product was mixed with the above-mentioned Hind III - Hind III DNA fragment containing the promoter region and signal peptide region of the penicillinase gene, and these fragments were ligated using T4 DNA ligase according to the same procedure as described in Example 2, and the reaction mixture was used to transform E. coli HB101, and from an ampicillin and chloramphenicol resistant transformant, a plasmid pPS1 of about 3.7 Kbp was isolated and purified. In this plasmid pPS1, the promoter region and signal peptide region of the penicillinase gene was inserted upstream from the CAT gene in the same reading orientation. Figure 7 represents the process for the construction of the plasmid pPS1.

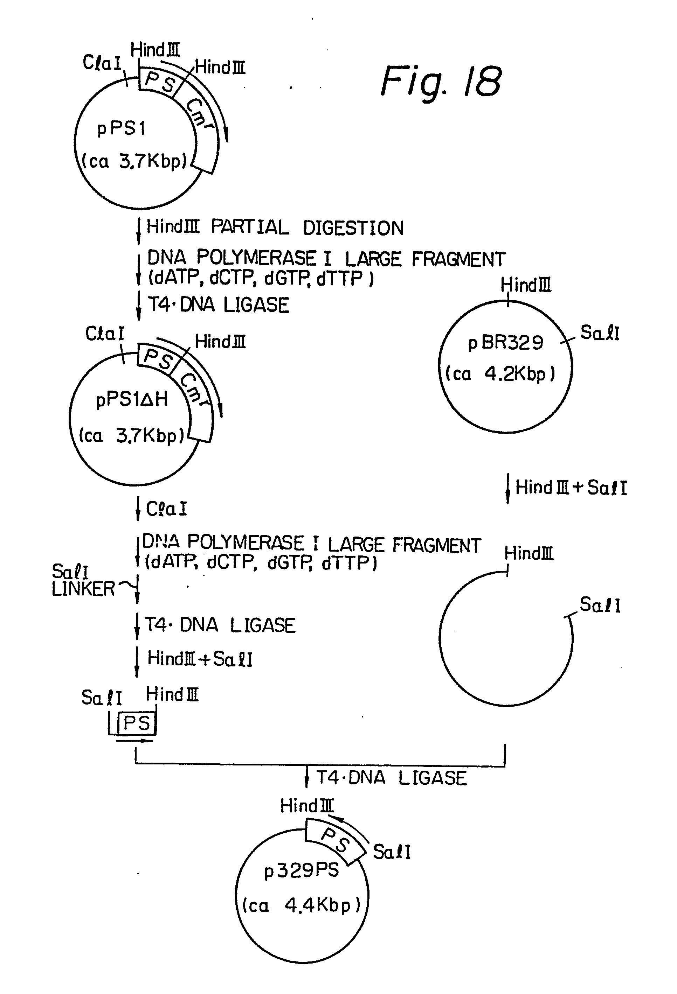

The plasmid pPS1 thus obtained was partially digested with the restriction enzyme Hind III according to the same procedure as described in Example 4, and after agarose gel electrophoresis (gel concentration 0.8%), a Hind III - Hind III DNA fragment of about 3.7 Kbp formed by cleavage of one Hind III site among the Hind III sites originally present was recovered in the same manner as described above. This Hind III - Hind III DNA fragment was blunt-ended using a DNA polymerase I large fragment according to the same procedure as described in Example 12, and then self-ligated using T4 DNA ligase according to the same procedure as described in Example 2. In such a manner, plasmid pPS1ΔH of about 3.7 Kbp was constructed wherein a Hind III site present upstream from the penicillinase gene promoter region was deleted. Figure 18 represents the process for the construction of the plasmid pPS1ΔH.

Moreover, plasmid pPS1ΔH was cleaved with restriction enzyme Cla I according to the same procedure as described in Example 4, and the product was mixed with an 5'-end-phosphorylated Sal I linker (Takara Shuzo), and ligation was carried out using T4 DNA ligase according to the same procedure as described in Example 2. After ethanol precipitation, the ligation product was cleaved with restriction enzymes Hind III and Sal I. Polyacrylamide gel electrophoresis (gel concentration 5%) was then carried out, and a Sal I - Hind III DNA fragment of about 210 bp containing the promoter region and signal peptide region of the penicillinase gene was recovered by electroelution.

On the other hand, plasmid pBR329 was cleaved with restriction enzymes Hind III and Sal I, and after agarose gel electrophoresis (gel concentration 0.8%), a Hind III - Sal I DNA fragment of about 3.6 Kbp containing the major vector portion was recovered. The two DNA fragments thus obtained were mixed, and ligated using T4 DNA ligase according to the same procedure as described in Example 2, and in the same manner as described above, plasmid p329PS was constructed. Figure 18 represents the process for the construction of the plasmid p329PS.

### Example 18. Construction of periplasmic transportation type plasmid for Fc region gene

The intracellular accumulation type plasmid pFC362 containing the Fc region gene, constructed in Example 11, was cleaved with restriction enzymes Sst II and Eco RI according to the same procedure as described in Example 4, and after an agarose gel electrophoresis (gel concentration 0.8%), an Eco RI - Sst II DNA fragment of about 0.4 Kbp containing the tac promoter region, hinge domain gene and 5'-terminal half of a $C_H2$ domain gene, and Eco RI - Sst II DNA fragment of about 4.5 Kbp containing a major vector portion, 3'-terminal half of $C_H2$ domain gene and $C_H3$ domain gene were recovered from the gel according to the same procedure as described in Example 5. Among these DNA fragments, the Eco RI - Sst II DNA fragment containing the tac promoter region, hinge domain gene, and 5'-terminal half of $C_H2$ domain gene was cleaved with restriction enzyme Bst NI according to the same procedure as described in Example 4, and after polyacrylamide gel electrophoresis (gel concentration 5%), Bst NI - Sst II DNA fragment of about 171 bp containing a portion of the $C_H2$ domain gene was recovered from the gel according to the same procedure as described in Example 7.

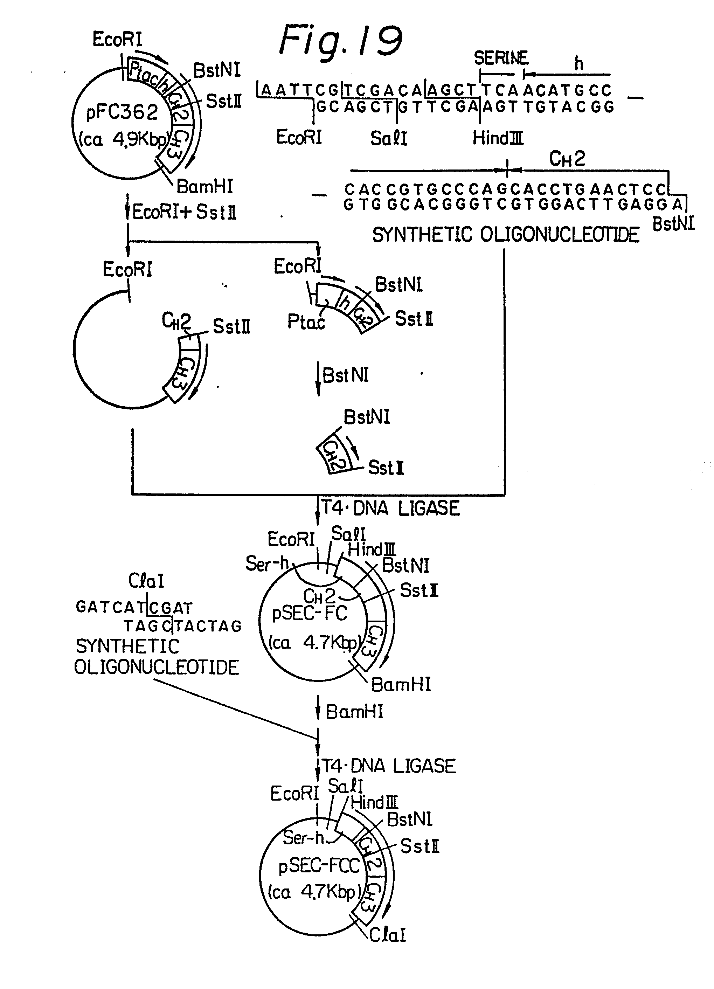

A double-stranded oligonucleotide of about 51 bp (including the protruding ends) having the nucleic acid sequence shown in Fig. 19 corresponding to a linking region between the penicillinase gene promoter / signal peptide region and Fc region gene was prepared according to the same procedure as described in Example 8. This Eco RI - Bst NI DNA fragment contained the restriction enzyme Sal I site necessary for subcloning in a later step, restriction enzyme Hind III site used for linking with the signal peptide region gene of the penicillinase gene, TCA codon coding for amino acid serine, hinge region gene and a portion of the $C_H2$ domain gene. By using this DNA fragment, it is expected that the signal peptide is correctly processed and the Fc region protein having an additional amino acid serine at its amino terminal is produced.

The Eco RI - Sst II DNA fragment containing a major vector portion, a 3'-terminal half of $C_H2$ domain gene and $C_H3$ domain gene, a Bst NI - Sst II DNA fragment containing a portion of the $C_H2$ domain gene, and an Eco RI - Bst NI DNA fragment corresponding to a linking region between a signal peptide region of the penicillinase gene and Fc region gene, all prepared as described above, were mixed, and plasmid pSEC-FC of about 4.7 Kbp was constructed according to the same procedure as described in Example 5. Figure 19 represents the process for the construction of the plasmid pSEC-FC.

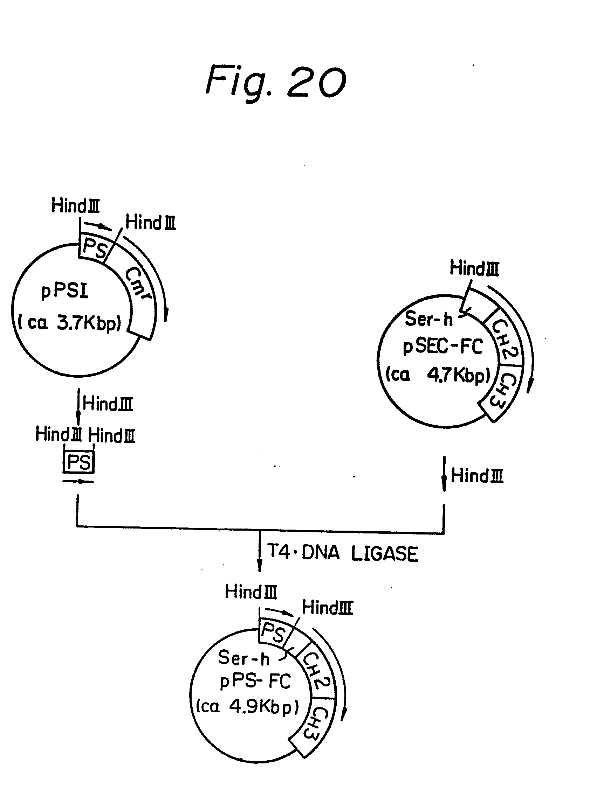

The plasmid pSEC-FC thus obtained was cleaved with restriction enzyme Hind III according to the same procedure as described in Example 4. On the other hand, plasmid pPS1 containing the promoter region and signal peptide region of the penicillinase gene, obtained in Example 17, was cleaved with restriction enzyme Hind III according to the same procedure as described in Example 4, and after polyacrylamide gel electrophoresis (gel concentration 5%), a Hind III - Hind III DNA fragment of about 210 bp containing the promoter region and signal peptide region of the penicillinase gene was recovered by electroelution method. This DNA fragment was mixed with the product above-prepared by cleaving plasmid pSEC-FC with restriction enzyme Hind III, and a periplasmic transportation type plasmid pPS-FC for the Fc region gene was constructed according to the same procedure as described in Example 17. Figure 20 represents the process for the construction of the plasmid pPS-FC.

### Example 19. Construction of extracellular secretion type plasmid for Fc region gene

The plasmid pSEC-FC constructed in Example 18 was cleaved with restriction enzyme Bam HI according to the same procedure as described in Example 4, and mixed with a double-stranded nucleotide of about 14 bp (including the protruding ends) having the nucleotide sequence shown in Fig. 19 and containing a restriction enzyme Cla I site and prepared according to the same procedure as described in Example 8, and ligation was carried out using T4 DNA ligase according to the same procedure as described in Example 2. According to the procedure described in Example 16, a restriction enzyme Bam HI site present in plasmid pSEC-FC was converted to the restriction enzyme Cla I site to construct a plasmid pSEC-FCC of about 4.7 Kbp. Figure 19 represents the process for the construction of the plasmid pSEC-FCC.

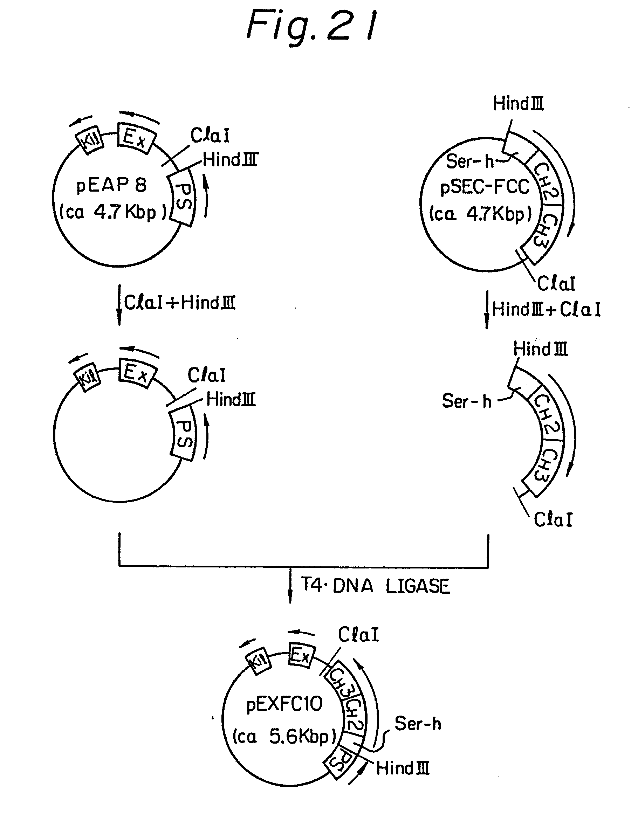

The plasmid pSEC-FCC thus obtained was cleaved with restriction enzymes Hind III and Cla I according to the same procedure as described in Example 4, and after agarose gel electrophoresis (gel concentration 0.8%), a Hind III - Cla I DNA of about 0.7 Kbp containing the Fc region gene was recovered as described above. On the other hand, the secretion type plasmid vector pEAP8 constructed in Example 16 was cleaved with restriction enzymes Hind III and Cla I, and after agarose gel electrophoresis (gel concentration 0.8%), a Cla I - Hind III DNA fragment of about 4.7 Kbp consisting of a major portion of the plasmid pEAP8 was recovered as described above. These two DNA fragments were mixed, and ligated using T4 DNA ligase according to the same procedure as described in Example 2, and an Fc region protein extracellular secretion type plasmid pEXFC10 of about 5.6 Kbp was constructed. Figure 21 represents the process for the construction of the plasmid pEXFC10.

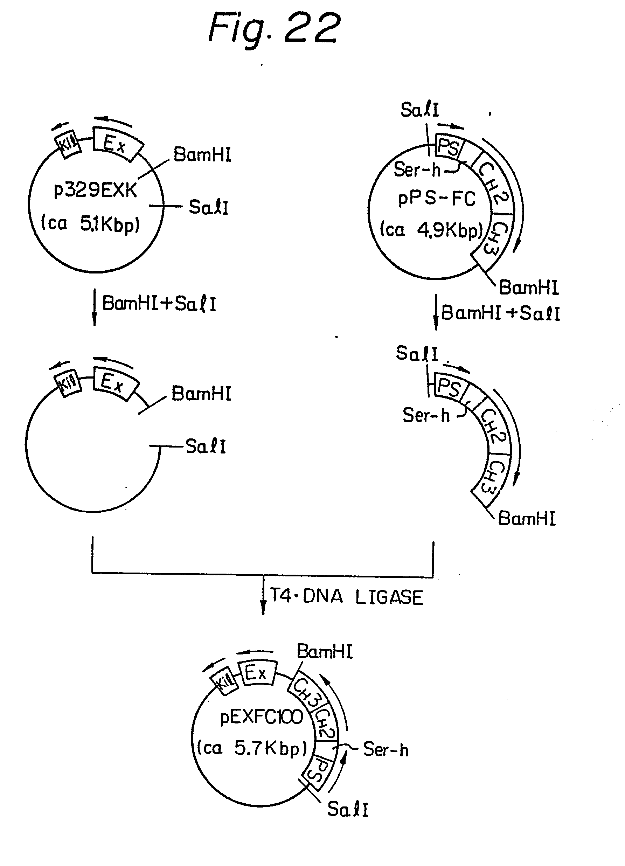

On the other hand, an Fc region protein periplasmic transportation type plasmid pPS-FC obtained in Example 18 was cleaved with restriction enzymes Bam HI and Sal I according to the same procedure described in Example 4, and after agarose gel electrophoresis (gel concentration 0.8%), an Sal I - Bam HI DNA fragment of about 0.9 Kbp containing a promoter region and signal peptide region of the penicillinase gene was recovered by an electroelution method. Moreover, a multi-copy secretion type plasmid vector p329EXK obtained in Example 16 was cleaved with restriction enzymes Bam HI and Sal I, and after agarose gel electrophoresis (gel concentration 0.8%), a Bam HI - Sal I DNA fragment of about 4.8 Kbp containing a major portion of the plasmid p329EXK was recovered. These two DNA fragments were ligated using T4 DNA ligase according to the same procedure as described in Example 2, and an Fc region protein extracellular secretion type plasmid pEXFC100 of about 5.7 Kbp was constructed according to the same procedure as described in Example 16. Figure 22 represents the process for the construction of the plasmid pEXFC100.

Example 20. Confirmation of periplasmic transportation and secretion of Fc region protein

E. coli C600R⁻M⁻ transformed with an Fc region protein intracellular accumulation type plasmid pFC362, obtained in Example 11; E. coli HB101 transformed with an Fc region protein periplasmic transportation type plasmid pPS-FC, obtained in Example 18; and E. coli HB101 transformed with an Fc region protein extracellular secretion type plasmid pEXFC10, and E. coli HB101 transformed with an Fc region protein extracellular secretion type plasmid pEXFC100, both obtained in Example 10 were inoculated into LBGG medium (1% trypton, 0.5% yeast extract, 1% NaCl, 0.1% glucose, 0.2% glycerol, pH 7.2) supplemented with 30 µg/ml of ampicillin, and culturing was carried out at 37°C for 24 hours, except that E. coli transformed with pEXFC10 was cultured in the same medium supplemented with 20 µg/ml of chloramphenicol in place of ampicillin.

After the culturing, each cultured broth was subjected to an osmotic shock method (C. Kato et al., supra) to separate it into an extracellular fraction, a periplasmic fraction, and an intracellular fraction. More specifically, the cultured broth was centrifuged to obtain cultured cells and a supernatant, which was designated as an extracellular fraction. Next, the cells thus collected were washed with a physiological saline, the washed cells were suspended in a 25% sucrose aqueous solution containing 1 mM EDTA, and the suspension was shaken for 10 minutes at 37°C. The suspension was centrifuged to collect the cells, which were then resuspended in ice-cooled water, and the suspension was shaken at 4°C for 10 minutes. To the suspension, the same volume of 0.1 M phosphate buffer (pH 7.0) was added. The mixture was centrifuged to separate cells from the supernatant, which was designated as the periplasmic fraction. The separated cells were suspended in a 0.05 M phosphate buffer (pH 7.0), and the cells were disrupted by ultrasonic waves. The treated suspension was centrifuged to eliminate cell debris from the supernatant, which was designated as an intracellular fraction.

An aliquot of each fraction was acetone-dried, and 60 mM Tris-HCl buffer (pH 6.8), 2% SDS, 4% 2-mercaptoethanol, and 10% glycerol was added to the dried product. The mixture was then subjected to SDS-polyacrylamide gel electrophoresis (Suzuki, Iden, 31, 43, 1977). The gel concentration of separation gel was 12.5%, and the migration buffer was SDS-Tris-glycine buffer (U.K.

Laemmli, *Nature*, 227, 680 (1970)). After the electrophoresis, proteins in the gel were electrophoretically transferred to and adsorbed on a nitrocellulose filter in a buffer containing 25 mM Tris, 192 mM glycine - (pH 8.3) and 20% methanol to carry out Western blotting.

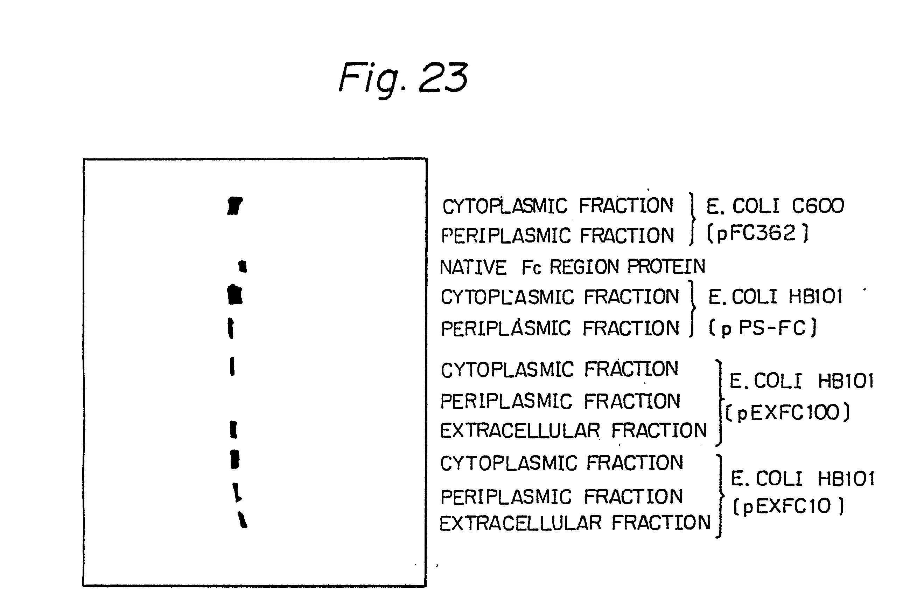

The nitrocellulose filter on which proteins has been adsorbed was soaked in PBS buffer containing 5% bovine serum albumin for 60 minutes, and then the human immunoglobulin G Fc region protein was specifically stained by an indirect method using, as a primary antibody, a rabbit anti-human IgG-Fc component antiserum (Cappel) employing an Immun-Blot assay kit (Bio Rad). The results are shown in Fig. 23.

In this confirmation procedure, a native human immunoglobulin G Fc region protein prepared by the procedure described hereinafter in the Reference Example was electrophoresed on the same SDS-polyacrylamide gel in parallel with the test samples, and the density of bands from test samples was compared with the density of the band of a known amount of the authentic (natural) sample to determine the amount of the former.

As seen from Fig. 23, when E. coli transformed with Fc region intracellular accumulation type plasmid pFC362 was cultured, the Fc region protein was localized in the intracellular fraction. On the other hand, when E. coli transformed with periplasmic transportation type plasmid pPS-FC was cultured, the Fc region protein was transported into the periplasmic space; and when E. coli transformed extracellular secretion type plasmid pEXFC10 or pEXFC100 was cultured, the Fc region protein was extracellularly secreted.

On the other hand, an aliquot of the above-mentioned extracellular fraction prepared by using an extracellular secretion type plasmid was acetone-dried, and 60 mM Tris-HCl (pH 6.8), 2% SDS and 10% glycerol was added to the dried product, and the mixture was subjected to SDS-polyacrylamide gel electrophoresis (gel concentration 12.5%).

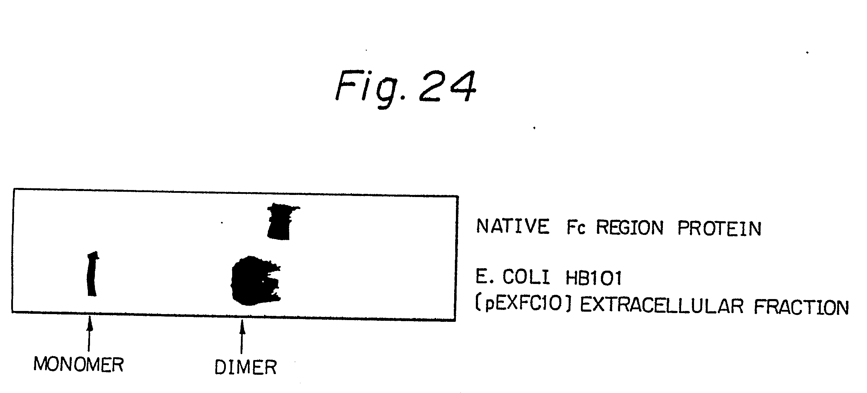

According to the same procedure as described above, the human immunoglobulin G Fc region protein was specifically stained. For comparison, a native human immunoglobulin G Fc region protein prepared in the Reference Example was treated in parallel. The results are shown in Fig. 24.

As seen from Fig. 24, the Fc region protein secreted by E. coli HB101 transformed with the Fc region protein extracellular secretion type plasmid pEXFC10 mainly comprises a dimer protein formed by disulfide bonds similar to the native Fc region protein.

Example 21. Purification of secreted Fc region protein

Three ml of an activated affinity carrier, Affigel 10 (Bio Rad) and 6.2 mg of an affinity-purified sheep anti-human IgG-Fc component antibody (Kappel) were coupled in 0.1 M MOPS buffer (pH 7.5; Nakarai Kagaku Yakuhin, Japan) to prepare an affinity column for purification of the Fc region protein. After coupling at 4°C for 2 hours, 40% of the sheep anti-human IgG-Fc component antibody used was immobilized onto the carrier.

An aliquot of the extracellular fraction prepared in Example 20 from culture of E. coli HB101 transformed with Fc region protein extracellular secretion type plasmid pEXFC10 was passed through the above-prepared affinity column to adsorb specifically the Fc region protein on the column. The column was sufficiently washed with a PBS buffer (100 mM phosphate buffer, pH 7.4, and 140 mM NaCl), and 20 mM phosphate buffer (pH 7.4) containing 500 mM NaCl, and then a 0.1 M glycine-HCl buffer (pH 2.3) was passed through the column to elute the Fc region protein. The eluted Fc region protein was dialyzed against water, and after lyophilization of the dialyzate, the product was subjected to SDS-acrylamide gel electrophoresis (gel concentration 12.5%). After the electrophoresis, protein bands in the gel were stained with a silver stain reagent (Daiichi Kagaku Yakuhin, Japan), and it was confirmed that the Fc region protein extracellularly secreted from E. coli was obtained at a purity of more than 98%.

Example 22. Analysis of secreted Fc region protein

The purified Fc region protein preparation prepared as described in Example 21 was analyzed for an amino-terminal amino acid sequence using a gas phase protein sequencer (Applied Biochemicals, Model 470A), and the following amino acid sequence:

$$H_2N\text{-Ser-Thr-(  )-Pro-Pro-(  )-Pro ---}$$

was shown. This result shows that the penicillinase signal peptide was correctly processed during the secretion.

Moreover, the secreted and purified Fc region protein preparation was hydrolyzed in 6 N HCl containing 2% thioglycolic acid at 110°C for 22 hours, and the hydrolysate was analyzed for amino acid composition using an amino acid analyzer (Hitachi 835). The results are shown in the following table.

0234592

| Amino acid | Calculated (Mol %) | Found (Mol %) |
|---|---|---|
| Asn/Asp | 8.93 | 8.84 |
| Thr | 6.70 | 6.16 |
| Ser | 9.36 | 8.82 |
| Gln/Glu | 11.16 | 11.83 |
| Gly | 4.46 | 5.12 |
| Ala | 3.12 | 3.59 |
| Val | 10.27 | 9.45 |
| Cys | 2.68 | 2.57 |
| Met | 1.34 | 1.41 |
| Ile | 1.79 | 1.88 |
| Leu | 7.59 | 7.84 |
| Tyr | 4.02 | 4.07 |
| Phe | 3.12 | 3.22 |
| Lys | 8.48 | 8.66 |
| His | 2.68 | 2.73 |
| Arg | 2.68 | 2.73 |
| Pro | 9.82 | 9.65 |
| Trp | 1.79 | 1.33 |
| Total | 100.00 | 100.00 |

It was found that the amino acid composition exactly conforms to that calculated from the nucleotide sequence of the Fc region gene, revealing that the Fc region protein secreted from E. coli has the intended amino - acid sequence. Moreover, because the molecular weight of the Fc region protein produced by E. coli is lower by about 5000 Dalton than that of the native Fc region protein, as shown in Fig. 23, it is speculated that the Fc region protein produced by E. coli has not been glycosylated.

Reference Example   Preparation of native human
immunoglobulin G Fc region
protein

0.3 g of human immunoglobulin G (Sigma), 17.5 mg of cysteine, and 7.2 mg of EDTA·2Na were dissolved in a PBS buffer (see Example 21), the solution was added with 150 µg of papain (Sigma Type IV), and allowed to stand for 7 hours at 37°C. The papain-treated IgG was applied on a gel filtration column containing Sephadex G-200 super fine gel equilibrated with PBS buffer to separate the Fc region protein and Fab region protein, both generated by the papain treatment, from intact IgG. An aqueous solution containing the Fc region protein and Fab region protein thus obtained was dialyzed against water, and the dialyzate was lyophilized. The lyophilized product was then applied to DE52·DEAE cellulose (Whatman) column for ion exchange chromatography. The column was washed with a 10 mM phosphate buffer (pH 7.4) to completely elute the Fab region protein, and then the Fc region protein was eluted by a linear gradient elution with from 0 mM to 350 mM NaCl in a 10 mM phosphate buffer (pH 7.4). Finally, dialysis and lyophilization were carried out as described above to obtain a natural type human immunoglobulin G Fc region protein.

0234592

## CLAIMS

1.    A plasmid containing a DNA region coding for a human immunoglobulin G Fc region protein, a DNA region exhibiting a promoter function for controlling expression of said protein, and a DNA region coding for a signal peptide.

2.    A plasmid according to claim 1 wherein said DNA region coding for said human immunoglobulin G Fc region protein contains at least a DNA region coding for a peptide having the following amino acid sequence:

                    Thr Cys Pro Pro Cys Pro Ala Pro Glu Leu
Leu Gly Gly Pro Ser Val Phe Leu Phe Pro Pro Lys Pro Lys
Asp Thr Leu Met Ile Ser Arg Thr Pro Glu Val Thr Cys Val
Val Val Asp Val Ser His Glu Asp Pro Glu Val Lys Phe Asn
Trp Tyr Val Asp Gly Val Glu Val His Asn Ala Lys Thr Lys
Pro Arg Glu Glu Gln Tyr Asn Ser Thr Tyr Arg Val Val Ser
Val Leu Thr Val Leu His Gln Asp Trp Leu Asn Gly Lys Glu
Tyr Lys Cys Lys Val Ser Asn Lys Ala Leu Pro Ala Pro Ile
Glu Lys Thr Ile Ser Lys Ala Lys Gly Gln Pro Arg Glu Pro
Gln Val Tyr Thr Leu Pro Pro Ser Arg Glu Glu Met Thr Lys
Asn Gln Val Ser Leu Thr Cys Leu Val Lys Gly Phe Tyr Pro
Ser Asp Ile Ala Val Glu Trp Glu Ser Asn Gly Gln Pro Glu
Asn Asn Thr Lys Thr Thr Pro Pro Val Leu Asp Ser Asp Gly
Ser Phe Phe Leu Tyr Ser Lys Leu Thr Val Asp Lys Ser Arg
Trp Gln Gln Gly Asn Val Phe Ser Cys Ser Val Met His Glu
Ala Leu His Asn His Tyr Thr Gln Lys Ser Leu Ser Leu Ser
Pro Gly Lys

3.    A plasmid according to claim 1, wherein said DNA region exhibiting said promoter function is derived from chromosomal DNA of an alkalophilic Bacillus strain No. 170.

4.    A plasmid according to claim 1, wherein said DNA region coding for said signal peptide is derived from chromosomal DNA of said alkalophilic Bacillus strain No. 170.

5.    A plasmid according to claim 1, wherein said plasmid is a plasmid pPS-FC.

6.    Microbial cells transformed with a plasmid according to claim 1.

7.    Microbial cells according to claim 6 which are of the genus *Escherichia*.

8.    Microbial cells according to claim 7 which are of *Escherichia coli* HB101.

9.    A process for production of human immunoglobulin G Fc region protein, comprising culturing said microbial cells according to claim 6 until the protein accumulates in a a periplasmic space, and recovering said human immunoglobulin G Fc region protein from the periplasmic space.

10.    A process according to claim 9 wherein said microbial cells are of the genus *Escherichia*.

11.    A process according to claim 10 wherein said microbial cells are of *Escherichia coli* HB101.

12.    A plasmid containing:

(a)    a DNA fragment comprising a DNA region coding for human immunoglobulin G Fc region protein, a DNA region exhibiting a promoter function for controlling expression of said protein, and a DNA region coding for a signal peptide; and

(b)    a DNA fragment comprising a DNA region causing host cells to facilitate extracellular secretion of said protein, and a DNA region exhibiting a promoter function for controlling expression of said DNA region.

13.    A plasmid according to claim 12 wherein the DNA region coding for human immunoglobulin G Fc region protein contains at least a DNA region coding for a peptide having the following amino acid sequence:

Thr Cys Pro Pro Cys Pro Ala Pro Glu Leu
Leu Gly Gly Pro Ser Val Phe Leu Phe Pro Pro Lys Pro Lys
Asp Thr Leu Met Ile Ser Arg Thr Pro Glu Val Thr Cys Val
Val Val Asp Val Ser His Glu Asp Pro Glu Val Lys Phe Asn
Trp Tyr Val Asp Gly Val Glu Val His Asn Ala Lys Thr Lys
Pro Arg Glu Glu Gln Tyr Asn Ser Thr Tyr Arg Val Val Ser

Val Leu Thr Val Leu His Gln Asp Trp Leu Asn Gly Lys Glu
Tyr Lys Cys Lys Val Ser Asn Lys Ala Leu Pro Ala Pro Ile
Glu Lys Thr Ile Ser Lys Ala Lys Gly Gln Pro Arg Glu Pro
Gln Val Tyr Thr Leu Pro Pro Ser Arg Glu Glu Met Thr Lys
Asn Gln Val Ser Leu Thr Cys Leu Val Lys Gly Phe Tyr Pro
Ser Asp Ile Ala Val Glu Trp Glu Ser Asn Gly Gln Pro Glu
Asn Asn Thr Lys Thr Thr Pro Pro Val Leu Asp Ser Asp Gly
Ser Phe Phe Leu Tyr Ser Lys Leu Thr Val Asp Lys Ser Arg
Trp Gln Gln Gly Asn Val Phe Ser Cys Ser Val Met His Glu
Ala Leu His Asn His Tyr Thr Gln Lys Ser Leu Ser Leu Ser
Pro Gly Lys

14. A plasmid according to claim 12 wherein said DNA region exhibiting the promoter function for controlling the expression of said protein is derived from chromosomal DNA of alkalophilic Bacillus strain No. 170.

15. A plasmid according to claim 12 wherein the DNA region coding for a signal peptide is derived from chromosomal DNA of alkalophilic Bacillus strain No. 170.

16. A plasmid according to claim 12 wherein the DNA region causing host cells to facilitate the extracellular secretion of the protein is derived from plasmid pMB9.

17. A plasmid according to claim 12 wherein the DNA region exhibiting the promoter function for controlling the expression of the DNA region in item (b) of claim 12 is derived from chromosomal DNA of alkalophilic Bacillus strain No. 170.

18. A plasmid according to claim 12, wherein said plasmid is a plasmid pEXFC10.

19. A plasmid according to claim 12, wherein said plasmid is plasmid pEXFC100.

20. Microbial cells transformed with a plasmid according to claim 12.

21. Microbial cells according to claim 20 which are of the genus Escherichia.

22. Microbial cells according to claim 21 which

are of <u>Escherichia coli</u> HB101.

23. A process for production of human immunoglobulin G Fc region protein comprising culturing microbial cells according to claim 20 until the protein accumulates outside the cells, and recovering the human immunoglobulin G Fc region protein from the cultured broth.

24. A process according to claim 23 wherein the microbial cells are of the genus <u>Escherichia</u>.

25. A process according to claim 24 wherein the microbial cells are of <u>Escherichia coli</u> HB101.

# Fig. I-I

ACGCTTTTTTTGTTAATTACATAAAAGTATGCAAA

TGAAGATGGAACAACATTTGAGATGAATTGTCTAATATAGGTAATAACTATTTAGC

TTGAAAGAAAGGGTTGATAACATG-AAA-AAG-AAT-ACG-TTG-TTA-AAA-GTA-
              Met-Lys-Lys-Asn-Thr-Leu-Leu-Lys-Val-
              (1)

GGA-TTA-TGT-GTA-AGT-TTA-CTA-GGA-ACA-ACT-CAA-TTT-GTT-AGC-
Gly-Leu-Cys-Val-Ser-Leu-Leu-Gly-Thr-Thr-Gln-Phe-Val-Ser-
(10)                                          (20)

              Hind III(10)            100
ACG-ATT-TCT-TCT-GTA-CAA-GCT-TCA-ACA-TGC-CCA-CCG-TGC-CCA-
Thr-Ile-Ser-Ser-Val-Gln-Ala-Ser-Thr-Cys-Pro-Pro-Cys-Pro-
              (30)

                                          150
GCA-CCT-GAA-CTC-CTG-GGG-GGA-CCG-TCA-GTC-TTC-CTC-TTC-CCC-
Ala-Pro-Glu-Leu-Leu-Gly-Gly-Pro-Ser-Val-Phe-Leu-Phe-Pro-
     (40)                                     (50)

CCA-AAA-CCC-AAG-GAC-ACC-CTC-ATG-ATC-TCC-CGG-ACC-CCT-GAG-
Pro-Lys-Pro-Lys-Asp-Thr-Leu-Met-Ile-Ser-Arg-Thr-Pro-Glu-
                        (60)

## Fig. 1-2

```
       200
GTC-ACA-TGC-GTG-GTG-GTG-GAC-GTG-AGC-CAC-GAA-GAC-CCT-GAG-
Val-Thr-Cys-Val-Val-Val-Asp-Val-Ser-His-Glu-Asp-Pro-Glu-
                    (70)


                 250
GTC-AAG-TTC-AAC-TGG-TAC·-GTG-GAC-GGC-GTG-GAG-GTG-CAT-AAT-
Val-Lys-Phe-Asn-Trp-Tyr-Val-Asp-Gly-Val-Glu-Val-His-Asn-
(80)                                                 (90)


                     300
GCC-AAG-ACA-AAG-CCG-CGG-GAG-GAG-CAG-TAC-AAC-AGC-ACG-TAC-
Ala-Lys-Thr-Lys-Pro-Arg-Glu-Glu-Gln-Tyr-Asn-Ser-Thr-Tyr-
                       (100)


                             350
CGG-GTG-GTC-AGC-GTC-CTC-ACC-GTC-CTG-CAC-CAG-GAC-TGG-CTG-
Arg-Val-Val-Ser-Val-Leu-Thr-Val-Leu-His-Gln-Asp-Trp-Leu-
          (110)                                   (120)


                                     400
AAT-GGC-AAG-GAG-TAC-AAG-TGC-AAG-GTC-TCC-AAC-AAA-GCC-CTC-
Asn-Gly-Lys-Glu-Tyr-Lys-Cys-Lys-Val-Ser-Asn-Lys-Ala-Leu-
                                 (130)


CCA-GCC-CCC-ATC-GAG-AAA-ACC-ATC-TCC-AAA-GCC-AAA-GGG-CAG-
Pro-Ala-Pro-Ile-Glu-Lys-Thr-Ile-Ser-Lys-Ala-Lys-Gly-Gln-
                       (140)
```

# Fig. 1-3

```
CCC-CGA-GAA-CCA-CAG-GTG-TAC-ACC-CTG-CCC-CCA-TCC-CGG-GAG-
Pro-Arg-Glu-Pro-Gln-Val-Tyr-Thr-Leu-Pro-Pro-Ser-Arg-Glu-
(150)                                                    (160)


GAG-ATG-ACC-AAG-AAC-CAG-GTC-AGC-CTG-ACC-TGC-CTG-GTC-AAA-
Glu-Met-Thr-Lys-Asn-Gln-Val-Ser-Leu-Thr-Cys-Leu-Val-Lys-
                            (170)


GGC-TTC-TAT-CCC-AGC-GAC-ATC-GCC-GTG-GAG-TGG-GAG-AGC-AAT-
Gly-Phe-Tyr-Pro-Ser-Asp-Ile-Ala-Val-Glu-Trp-Glu-Ser-Asn-
        (180)                                           (190)


GGG-CAG-CCG-GAG-AAC-AAC-TAC-AAG-ACC-ACG-CCT-CCC-GTG-CTG-
Gly-Gln-Pro-Glu-Asn-Asn-Tyr-Lys-Thr-Thr-Pro-Pro-Val-Leu-
                                (200)


GAC-TCC-GAC-GGC-TCC-TTC-TTC-CTC-TAT-AGC-AAG-CTC-ACC-GTG-
Asp-Ser-Asp-Gly-Ser-Phe-Phe-Leu-Tyr-Ser-Lys-Leu-Thr-Val-
                    (210)


GAC-AAG-AGC-AGG-TGG-CAG-CAG-GGG-AAC-GTC-TTC-TCA-TGC-TCC-
Asp-Lys-Ser-Arg-Trp-Gln-Gln-Gly-Asn-Val-Phe-Ser-Cys-Ser-
(220)                                                   (230)
```

0234592

# Fig. 1-4

```
700
GTG-ATG-CAT-GAG-GCT-CTG-CAC-AAC-CAC-TAC-ACG-CAG-AAG-AGC-
Val-Met-His-Glu-Ala-Leu-His-Asn-His-Tyr-Thr-Gln-Lys-Ser-
                              (240)

          750                            BamH I
                                           |
CTC-TCC-CTG-TCC-CCG-GGT-AAA-TAATAGGATCC
Leu-Ser-Leu-Ser-Pro-Gly-Lys
        (250)
```

# Fig. 2

Kil Gene

ATGACGAAAAGATTTTTTGTGGGAATATTCGCGATAAACCTCCTTGTTGGATGTCAGGCTAACTATATACGTGATGTTCAGGGAGGGACC
MetArgLysArgPhePheValGlyIlePheAlaIleAsnLeuLeuValGlyCysGlnAlaAsnTyrIleArgAspValGlnGlyGlyThr

ATCGCACCATCCTCCTCTTCTAAACTGACGGGGATCGCGGTTCAGTAG
IleAlaProSerSerSerSerLysLeuThrGlyIleAlaValGln***

# Fig. 3

Ex Promotor

```
Hinc II                                                 -35EcoRV        60
GTCAACAATA TGAACTGTCA CAAATCTTAT ATATATATTG TGA[TTGATA]T CACATCACTT

              -10           ┌─► mRNA                                   120
TTTTTCAATG GC[TATTAT]GC TTAAGGTGTA ATGAATGATT GGGAGAGGGT GGGATGATAT

                                                                       180
GTTTTGTTAT CAATGTGAAC AAAGCCAACA GGCGGTTGTA AAGTAATGGG TGTTTGTGGC

                                                                       240
GAAGAATGAA ACGATTGCGA GTTTACAAGA TACGATTGTG TTTGGGCTAA AAGGAATTGC

                                                                       300
AGCTTATCGC ACACATGCTG CTCAGCTAGG GTATACGGAT GCATTTGTAG ATGCTACAAC

  Hind III 311
ACAAGAAGCT T ────────[Kil Gene]────────
                                  ────►
```

## Fig. 4A

EcoRI-(1)  HindⅢ-(1)        HindⅢ-(2)                    HindⅢ-(3)  EcoRI-(2)

CHARON 4A
LEFT ARM | ca 3.4Kbp | ca 3.6Kbp | | XboI | ca 5.8Kbp | CHARON 4A
RIGHT ARM

XboI                    XhoI
ca 3.4Kbp | ca 3.6Kbp | ca 8.2 Kbp | ca 5.8Kbp

## Fig. 4B

HindⅢ-(2)
PstI-(2)

PstI-(3)

pTJ1B
(ca 12.6Kbp)

HindⅢ-(3)
XhoI

## Fig. 4C

SmaI-(1)      PstI-(2)
SmaI-(2)
SmaI-(3)      pTJ5
(ca 6.1Kbp)
SmaI-(4)

PstI-(3)

0234592

# Fig. 5

# Fig. 6

TaqI
├──── C$_H$2 ────────────────────────────
CGAGAAAACCATCTCCAAAGCCAAAGGGCAGCCC
TCTTTTGGTAGAGGTTTCGGTTTCCCGTCGGG

SmaI-(1)
──────────────── C$_H$3 ────────→
CGAGAACCACAGGTGTACACCCTGCCCCCATCCC
GCTCTTGGTGTCCACATGTGGGACGGGGGTAGGG

SYNTHETIC OLIGONUCLEOTIDE

pFC70
C$_H$3 SmaI-(1)
SmaI-(2)

↓ SmaI PARTIAL DIGESTION
↓ BamHI

BamHI
C$_H$3 SmaI-(1)
SmaI-(2)

Sau3AI
PstI-(2)  TaqI              PstI-(3)
[h] [C$_H$2] [C$_H$3] ┄┄┄ ┤

↓ Sau3AI
↓ TaqI

Sau3AI TaqI
[C$_H$2]

T4 DNA LIGASE

Sau3AI/(BamHI)
C$_H$2  TaqI
pFC77      SmaI-(1)
(ca 3.9Kbp)  C$_H$3
SmaI-(2)

Fig. 7

SYNTHETIC OLIGO-NUCLEOTIDE

ClaI   trp PROMOTOR   START CODON   h   CH2   BstNI-(5)

CGATAATG ACATGCCCACCGTGCCCAGCACCTGAACTCC
TATTACTGTACGGGTGGCACGGGTCGTGGACTTGAGGA

10/30

0234592

# Fig. 8

PstI   BamHI

pBR322

↓ BamHI

↓ PstI

PstI   BamHI

SmaI-(2)          BamHI
        STOP CODON

```
GGGTAAATAATAG
CCCATTTATTATCCTAG
```
SYNTHETIC OLIGONUCLEOTIDE

↓ T4-DNA LIGASE

PstI
pFC211
(ca 5.0Kbp)
Ptrp h CH2
CH3 ─ SmaI-(1)
SmaI-(2)
BamHI

PstI   Ptrp h CH2 CH3
pFC203
SmaI-(1)
SmaI-(2)

↓ SmaI PARTIAL DIGESTION

↓ PstI

PstI   Ptrp h CH2 CH3
SmaI-(1)
SmaI-(2)

11/30

0234592

Fig. 9

# Fig. 10-1

Cells

    Suspending in 10 ml of solution containing 20% sucrose, 50 mM Tris-HCl (pH 8.0) and
    1 mM EDTA on ice bath.

50 ml Polypropylene Centrifuge Tube

    Adding 2 ml of 0.25 M EDTA.

    Adding 1 ml of Lysozyme [5 mg/ml, 0.025 M Tris-HCl (pH 8.0)].

    Adding 0.1 ml of Ribonuclease A (10 mg/ml).

Lysis

    Mixing gently and standing for 10 to 30 min. in ice bath.  Adding 5 ml 3X Triton X-100
    and gently mixing and standing for 15 to 45 min. on ice bath.

Centrifugation at 17,000 r.p.m., 4°C, for 40 min.

Supernatant

250 ml Glass bottle

        Twice adding 2/3 volume of Distilled water.

        Adding 2/3 volume of cold water-saturated phenol and mixing gently.

0234592

# Fig. 10-2

Centrifugation at 6,500 r.p.m., 4°C, for 15 min.

Supernatant

     Adding equal volume of phenol/chloroform.

Centrifugation at 6,500 r.p.m., 4°C, for 15 min.

Supernatent

     Adding 1/25 volume of 5 M NaCl.

     Adding two volumes of ethanol and standing at -20°C over night.

Centrifugation at 6,500 r.p.m., -20°C, for 60 min.

DNA Pellet,

     Eliminating excessive liquid

     Redissolving in 5 ml of A-50 buffer.

     Adding 5 ml of sterilized 80% glycerol and gently mixing.

A-50 column (2 x 35 cm, 1 fraction = 4 ml)

0234592

# Fig. 10-3

DNA Fraction ($A_{260}$ peak).

       Adding two times of ethanol and standing at -20°C over night.

Centrifugation at 6,500 r.p.m., -20°C, for 60 min.

DNA Pellet

       Adding 2.1 ml of TEN Buffer [20 mM Tris-HCl (pH 7.5), 50 mM NaCl, 1 mM EDTA] (in 5 ml nitrocellulose tube).

       Adding 2.2 g of CsCl and mixing.

       Keeping in dark.

       Adding 150 µl of PbI (2 mg/ml) and completely mixing.

       Adding 2 ml of mineral oil.

CsCl Gradient centrifugation at 36,000 r.p.m., 20°C for 40 hrs.

       Observation by UV irradiation

           Upper band:  Chromosomal nicked DNA
           Lower band:  Covalently closed plasmid DNA

Collecting dropwise lower band DNA

# Fig. 10-4

Dowex 50W-X8 column (detection by UV)

Keeping in light.

Dialysis in 2 to 4 ℓ of buffer containing Tris-HCl (pH 8.0) and 1 mM EDTA at 4°C over night

30 ml Cortex centrifuge tube

Adding 1/25 volume of 5 M NaCl.

Adding 2 volume of ethanol and standing at -20°C over night.

Centrifugation at 6,500 r.p.m., -20°C, for 60 min.

DNA Precipitation

Adding 1 to 2 ml of TEN buffer.

Purified plasmid DNA (1 mg/1 ml culture) storage at -20 to -70°C.

# Fig. 11

EcoRI

pMB9
(ca 5.3Kbp)

ALKALOPHILIC BACILLUS No.170
CHROMOSOMAL DNA

↓ EcoRI          ↓ EcoRI

T4-DNA LIGASE

EcoRI
HindⅢ
HincⅡ

pEAP1
(ca 9.8Kbp)

Pen

HindⅢ

HindⅢ

HindⅢ
EcoRI

# Fig. 12

Fig. 13

# Fig. 14

# Fig. 15

# Fig. 16

## Fig. 17

Fig. 18

Fig. 19

# Fig. 20

# Fig. 21

# Fig. 22

Fig. 23

CYTOPLASMIC FRACTION  } E. COLI C600
PERIPLASMIC FRACTION  } (pFC362)

NATIVE Fc REGION PROTEIN

CYTOPLASMIC FRACTION  } E. COLI HB101
PERIPLASMIC FRACTION  } (p PS-FC)

CYTOPLASMIC FRACTION
PERIPLASMIC FRACTION     } E. COLI HB101
EXTRACELLULAR FRACTION   } (pEXFC100)

CYTOPLASMIC FRACTION
PERIPLASMIC FRACTION     } E. COLI HB101
EXTRACELLULAR FRACTION   } (pEXFC10)

# Fig. 24

NATIVE Fc REGION PROTEIN

E. COLI HB1O1
(pEXFC1O) EXTRACELLULAR FRACTION

↑
MONOMER          ↑
                 DIMER

| | DOCUMENTS CONSIDERED TO BE RELEVANT | | EP 87102830.4 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | EP - A2 - 0 088 994 (SCHERING CORPORATION)<br>* Claims 1-4,11 *<br>-- | 1,6,7,<br>9,10,<br>20,21,<br>23,24 | C 12 N 15/00<br>C 12 N 5/00<br>C 12 P 21/00<br>//(C 12 N 15/00;<br>C 12 R 1:07)<br>(C 12 N 5/00;<br>C 12 R 1:19) |
| D,A | THE EMBO JOURNAL, vol. 1, 1982, (Oxford, GB)<br>U. KRAWINKEL et al. "Comparison of the hinge-coding segements in human immunoglobulin gamma heavy chain genes and the linkage of the gamma 2 and gamma 4 subclass genes"<br>pages 403-407<br>* Totality *<br>-- | 1,12 | |
| D,A | NUCLEIC ACIDS RESEARCH, vol. 10, no. 13, 1982 (Oxford, GB)<br>J. W. ELLISON et al. "The nucleotide sequence of a human immunoglobulin $C_{\gamma_1}$ gene"<br>pages 4071-4079<br>* Totality *<br>-- | 1,12 | TECHNICAL FIELDS SEARCHED (Int Cl 4)<br>C 12 N<br>C 12 P |
| P,A | EP - A2 - 0 184 187 (TEIJIN LIMITED)<br>* Claims 1,3,4,9 *<br>-- | 1,12 | |
| A | EP - A2 - 0 107 509 (REPLIGEN CORPORATION)<br>* Abstract *<br>-- | 1,12 | |

The present search report has been drawn up for all claims

| Place of search | Date of completion of the search | Examiner |
|---|---|---|
| VIENNA | 03-06-1987 | WOLF |

Application number

European Patent Office

**EUROPEAN SEARCH REPORT**

| | DOCUMENTS CONSIDERED TO BE RELEVANT | | EP 87102830.4 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | EP - A1 - 0 125 023 (GENENTECH, INC; CITY OF HOPE)<br><br>* Abstract *<br><br>---- | 1,12 | |
| | | | **TECHNICAL FIELDS SEARCHED (Int Cl 4)** |

The present search report has been drawn up for all claims

| Place of search | Date of completion of the search | Examiner |
|---|---|---|
| VIENNA | 03-06-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**
FERM BP-469